(19) Europäisches Patentamt
European Patent Office
Office européen des brevets

(11) Publication number: **0 008 867**
**B1**

(12) **EUROPEAN PATENT SPECIFICATION**

(45) Date of publication of patent specification: **13.04.83**

(21) Application number: **79301513.2**

(22) Date of filing: **30.07.79**

(51) Int. Cl.³: **A 01 N 53/00,**
C 07 C 121/70,
C 07 C 121/75,
C 07 C 120/00,
C 07 D 317/60,
C 07 D 333/24
//C07C69/757, C07C67/313

(54) **Cyanovinyl pyrethroids, processes for the preparation of these compounds and pesticidal use of the cyanovinyl pyrethroids.**

(30) Priority: **28.08.78 US 937359**
**28.08.78 US 937360**

(43) Date of publication of application:
**19.03.80 Bulletin 80/6**

(45) Publication of the grant of the patent:
**13.04.83 Bulletin 83/15**

(84) Designated Contracting States:
**AT BE CH DE FR GB IT NL SE**

(56) References cited:
**FR - A - 2 362 588**

(73) Proprietor: **AMERICAN CYANAMID COMPANY**
**Berdan Avenue**
**Wayne New Jersey 06904 (US)**

(72) Inventor: **Brown, Dale Gordon**
**Stony Brook Road**
**Hopewell, New Jersey (US)**

(74) Representative: **Allam, Peter Clerk et al,**
**LLOYD WISE, TREGEAR & CO. Norman House**
**105-109 Strand**
**London WC2R 0AE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 008 867 B1

Cyanovinyl pyrethroids, processes for the preparation of these compounds, and pesticidal use of the cyanovinyl pyrethroids

This invention relates to a method for the control of ticks which are insect pests of agriculturally important crops and ectoparasites of domesticated warm-blooded animals.

In accordance with this invention there is provided a method for the control of ticks which comprises contacting the insects or ectoparasites, or applying to their hosts or to their habitat, a compound of the formula:

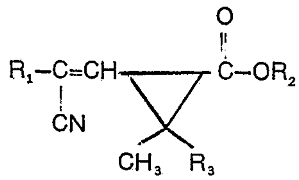
—(I)

wherein $R_1$ is 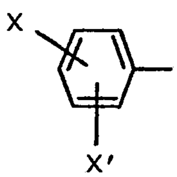 , 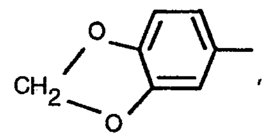 , 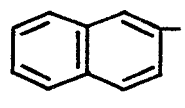 or 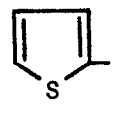 ;

$R_2$ is a moiety 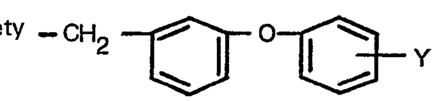 or 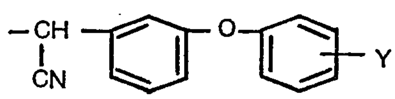

$R_3$ is hydrogen or methyl; X and X′ are each hydrogen, halogen, $C_1$—$C_3$ alkyl, $C_1$—$C_3$ alkoxy or haloalkyl $C_1$—$C_2$; Y is hydrogen, halogen, methyl or methoxy; in amounts sufficient to achieve control of the ticks.

Control of the insect pests of crops may be achieved by applying an effective amount of a compound of formula (I) above to the foliage and stems of the crops and/or to the soil in which these crops propagate and grow. Control of the ectoparasites, especially acarina, can be best achieved by contacting the ectoparasites with an effective amount of a compound of the invention or by applying an effective amount of the compound topically to the host animal and/or treating their environment with same. Alternatively, these compounds may be used for systemic control of the ectoparasites, especially acarina, by administering to the host animal orally or parenterally an effective amount of the substituted cyclopropanecarboxylic acid esters of formula (I). It will be recognized, of course, that the compounds represented by formula (I) above may exist in a number of geometric and optical isomeric forms, and mixtures thereof, and that varying degrees of insecticidal and acaricidal activity would be expected to be associated with each isomer. All such isomeric forms, and mixtures thereof, are within the scope of the present invention. Those isomers in which the carboxylic acid (ester) and the $\beta$-cyanostyryl functions are in the opposite sides of the cyclopropane ring are designated as *trans*; those in which they are on the same side are designated as *cis*. In each case, these may be separated into (+) and (—) optical isomers by appropriate methods, such as, for instance, the use of chiral bases in the separation of the carboxylic acid precursors (of formula IV below) of the above formula (I) compounds. A further locale for geometrical isomerism is the $\beta$-cyanostyryl function itself. In this case the designation *Z* is given to those compounds in which the cyano and cyclopropyl substituents are on the same side of the carbon-carbon double bond, whereas the *E* isomer is that in which the aryl or heterocyclic and the cyclopropyl substituents are so situated.

When the formula IV acids are esterified with $\alpha$-cyano-*m*-phenoxybenzyl alcohol, the resulting formula (I) esters have an additional chiral center. It is recognized that certain molecular species combining special combinations of these geometrical and optical characteristics will possess superior insecticidal and acaricidal activity. The invention includes all such combinations. Thus, the hereinabove defined compounds of formula (I) may be the *cis* and *trans* cyclopropane isomers, the *E* and *Z* side-chain olefin isomers, the optical isomers thereof and the isomeric mixtures thereof.

A preferred group of compounds for tick control represented by formula (I) above are those wherein $R_1$ is selected from

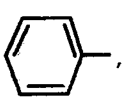 , 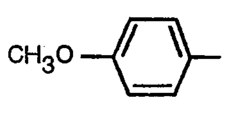 , 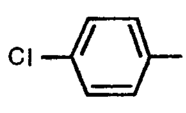 , 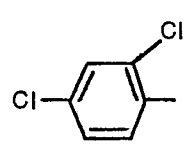 , 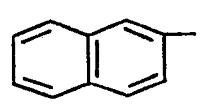 and 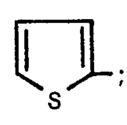 ;

$R_2$ is selected from 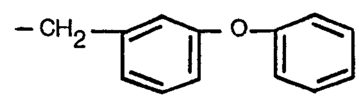 and 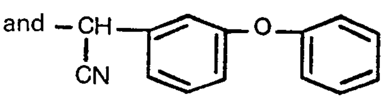 ;

$R_3$ is methyl; the *cis* and *trans* cyclopropane isomers, the *E* and *Z* side-chain olefin isomers, the optical isomers thereof and the isomeric mixtures thereof.

Novel compounds of the invention are compounds of the formula

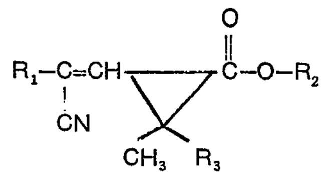

wherein $R_1$ is 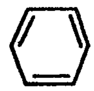 , 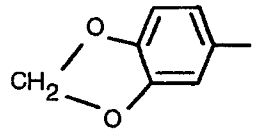 , 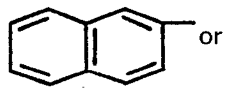 or 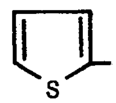 ;

$R_2$ is 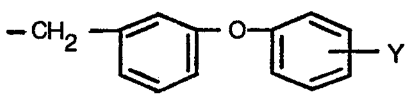 or 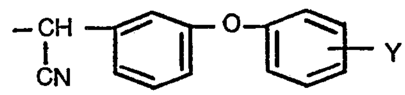

$R_3$ is hydrogen or methyl; and Y is hydrogen, halogen, methyl or methoxy.

Among the economic losses caused annually by acarina, those attributable to the sub-order Ixodidae are of considerable importance. Ixodid ticks are responsible for the maintenance and propagation of a great many human and animal diseases throughout the world. They are vectors for the diseases: tick paralysis and tick toxicosis. A single tick species can cause paralysis of several different mammals, and several tick species can cause paralysis in a particular host. Tick-borne diseases, such as Sweating Sickness, Babesiosis, Anaplasmosis. Thieleriosis and Heartwater, have been and are responsible for the death and/or debilitation of a vast number of animals throughout the world each year. In point of fact, of all external parasites, ticks are responsible for the greatest economic losses in livestock production in the world today. Such losses are, of course, attributed not only to death, but also damaged hides, loss in growth rate, reduction in milk production and reduced grade of meat animals.

The hereinabove described and defined compounds represented by formula (I) are eminently suitable for the control of Ixodidae. Control is effected by means of ixodicidal and of chemosterilant activity which the formula (I) compounds exert upon the larvae, nymphs, and adult male and female ticks. This method of control is useful against Argasidae or Ixodidae ticks, including, for example, those of the following types: *Boophilus, Amblyomma, Anocentor, Dermacentor, Ixodes, Haemaphysalis, Hyalomma, Rhipicentor, Margaropus, Rhipicephalus, Argas, Otobius* and *Ornithodorus.*

Conveniently, the compounds represented by formula (I) may be prepared by the following reaction sequence:

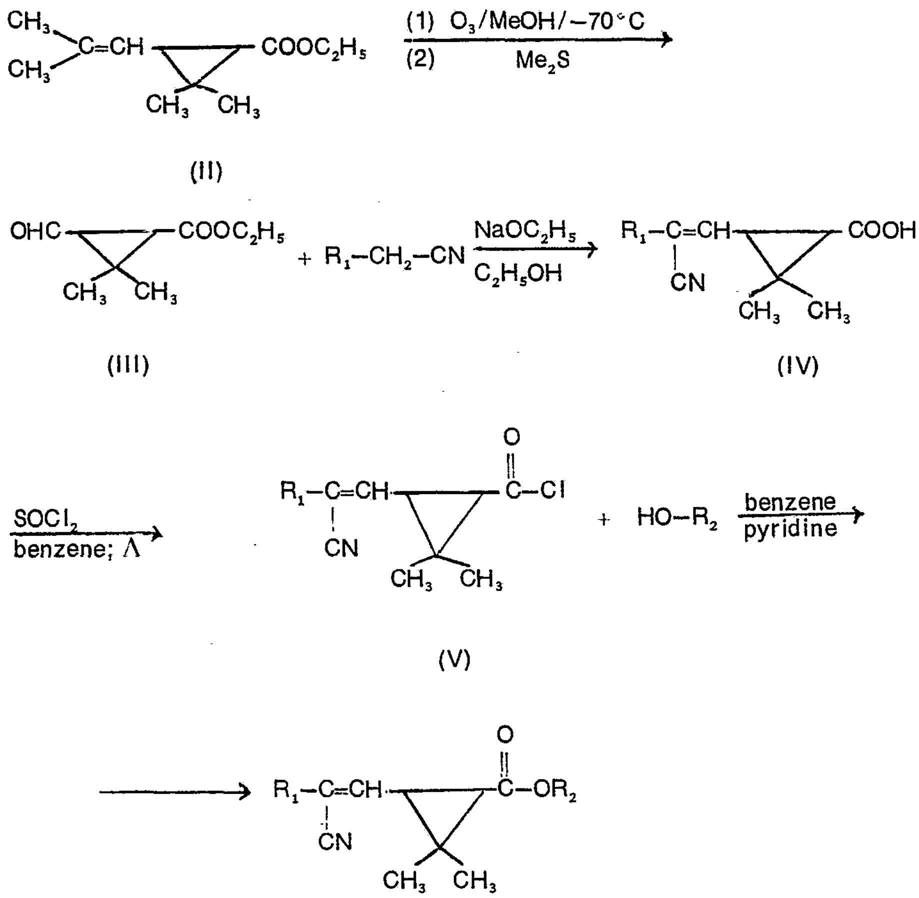


(II)

(III)

(IV)

(V)

(I)

wherein in the above reaction sequence $R_1$ and $R_2$ are as hereinbefore defined and $R_3$ was chosen to represent $CH_3$—.

Thus, ethyl chrysanthemate (II), wherein $R_3$ is methyl, is oxidized with ozone in a methanolic solution at −50 to −70°C to yield the corresponding 3-formyl-2,2-dimethylcyclopropanecarboxylic acid ethyl ester (III). Next, the thus obtained formyl compound is condensed in the presence of a base, such as sodium ethoxide in a lower alcohol with a compound of formula: $R_1$—$CH_2$—CN having an active methylene group to yield a cyclopropanecarboxylic acid of formula (IV). This acid is converted to the corresponding acid chloride (V) with thionyl chloride in an inert aromatic solvent such as benzene, toluene or xylene. The acid chloride (V) is then reacted with an alcohol of formula $R_2$—OH in the presence of an acid acceptor, such as pyridine, in an inert aromatic solvent such as benzene, toluene or xylene to afford the desired cyclopropanecarboxylic esters of formula (I).

Alternatively, formula (I) compounds may be prepared from the corresponding cyclopropanecarboxylic acids via ester forming reactions promoted by phase transfer catalysts. The above referred-to reactions may be schematically illustrated as follows:

A

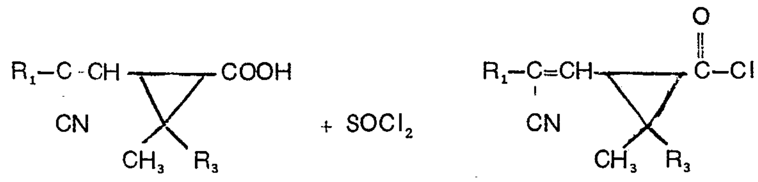


(IV) (V)

V + OHC–$R_4$+MCN/$H_2O$ 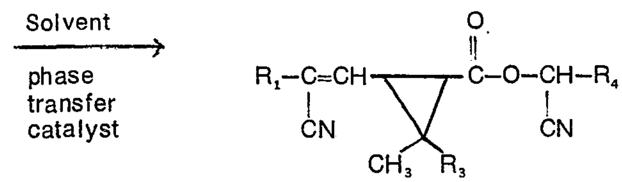


and

B

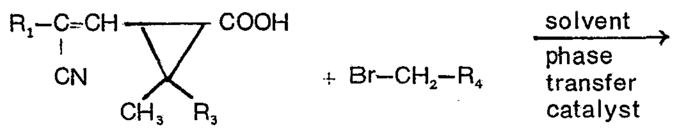


(IV)

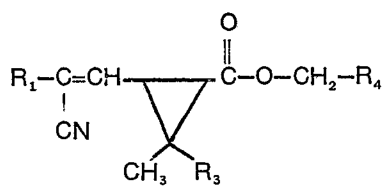

(I)

wherein in the above reaction schemes *A* and *B*, $R_1$ is selected from

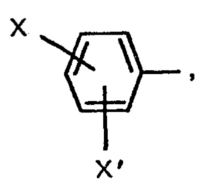, 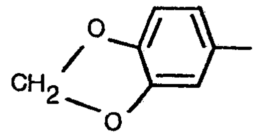, 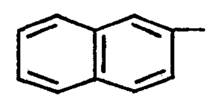 and 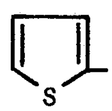 ;

$R_3$ is selected from hydrogen or methyl; $R_4$ is 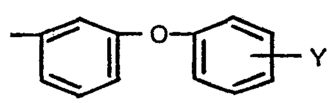

M is sodium or potassium; X and X′ are each selected from hydrogen, halogen, $C_1$—$C_3$ alkyl, $C_1$—$C_3$ alkoxy and haloalkyl $C_1$—$C_2$; Y is selected from hydrogen, halogen, methyl or methoxy; the *cis* and *trans* cyclopropane isomers, the *E* and *Z* side-chain olefin isomers the optical isomers thereof and the isomeric mixtures thereof.

In the above reactions, the phase transfer catalysts are selected from benzyltriethylammonium chloride, benzyltri-*n*-propylammonium chloride, $\alpha$-methylbenzyltriethylammonium iodide, tetrabutylammonium chloride and iodide, methyl tricaprylammonium chloride, hexadecyl trimethylammonium bromide, benzyl triphenylphosphonium chloride, hexadecyl tributyl phosphonium bromide, crown ethers, such as 18-crown-6, dicyclohexyl-18-crown-6 and dibenzo-18-crown-6. These phase transfer catalysts are utilized in amounts of 0.1% to 10% molar quantities and preferably 5% to 10% molar quantities. Solvents for these reactions are selected from methylene chloride, chloroform, 1,2-dichloroethane, hexane, heptane cyclohexane, benzene, toluene, xylene and ethyl acetate. The above described reactions are further illustrated in the Examples appended hereto.

Control of Ixodids is achieved by contacting the larvae, nymphs and the adult males and females with the cyclopropanecarboxylic esters for formula (I). Application can be made directly or indirectly. It is generally affected by topically applying the active ingredients, namely the compounds of formula (I) onto the host to be protected or to the habitat of the Ixodid.

Application is generally facilitated by employing a composition containing an effective amount of a compound of formula (I) in combination with an inert agricultural adjuvant. One or more of the conventional solid or liquid carriers, diluents and formulation aids may be employed as the adjuvant. Furthermore, in addition to employing a single compound of formula (I) as the active ingredient, several of the formula (I) compounds, or one or more of the compounds in combination with conventional pesticides may be employed.

The compounds of the invention as represented by formula (I) may be conveniently formulated as dusts, dust concentrates, wettable powders, emulsifiable concentrates, and the like. Application thereof is made in conventional manners, such as, by spraying, dusting, dipping in baths, and the like.

Solid formulations such as dusts, dust concentrates, can be prepared by grinding and blending together an inert solid diluent such as attapulgite, kaolin, walnut shell flour, diatomaceous earth, ground corncob grits, or ground coconut shell, and the active ingredient where such ingredient is in solid form. Where the active ingredient is a liquid, it may be sprayed on the carrier and thoroughly mixed therewith, or it may be dissolved in a solvent such as acetone, lower alkanol, toluene, xylene and the like, and sprayed as a dilute solution on the solid carrier. Dusts usually contain from about 1% to 15% by weight of active ingredient, whereas concentrates may contain from about 16% to about 85% by weight of the active material.

Wettable powders are prepared in the same fashion as dust concentrates excepting that about 5% to 10% by weight of a surfactant is also added. The wettable powder is then generally dispersed in water, or other suitable diluent for application as a dilute spray onto the Ixodid, host or locus where control is desired or as a bath for dipping animal hosts.

The formula (I) cyclopropanecarboxylic acid esters may also be prepared as emulsifiable concentrates by dissolving or dispersing about 10% to 75% by weight of the active compound in a suitable solvent carrier such as a petroleum distillate having a minimum aromatic content of 85%, and admixing therewith about 10% by weight of an emulsifier such as polyoxyethylene condensates and blends of same with alkyl aryl sulfonates. These concentrates are also generally dispersed in water or other suitable solvent for application by spraying or dipping the animal host.

Application of the formula (I) compounds at rates in the range of from about 1 ppm to 250 ppm for the control of ectoparasites, and especially Ixodidae, is generally preferred.

For the control of insect pests of agriculturally important crops application at rates in the range of from about 10 ppm to 1000 ppm is generally preferred.

The following non-limiting Examples further serve to illustrate the invention.

## Example 1

Preparation of 3-formyl-2,2-dimethyl-cyclopropanecarboxylic acid, ethyl ester

A solution of ethyl chrysanthemate (156.0 g) in absolute methanol (1400 ml) is stirred, cooled to −65°C, and ozone bubbled in at a rate of 1.7 l/min. under 0.56 kg/cm$^2$ pressure (the ozone is generated with a Welsbach T—23 ozonator; voltage setting 120 VAC). During the reaction, the temperature range of the reaction mixture fluctuates between −65°C and −50°C.

The reaction is followed with gas chromatography [6′ × 1/4″ glass column, packed with 3% OV—1 on WHP; temperature 130°C; sample size 0.2$\lambda$; flow 45 ml/min He; FID detector] to measure the disappearance of ethyl chrysanthemate. The ozonolysis is stopped after about 6 to 7 hours when about 5% ethyl chrysanthemate is still present. Methyl sulfide (125 ml) is added over 20 minutes, and the reaction mixture allowed to warm up to room temperature overnight with stirring. Next, the methanol is removed *in vacuo* at 50—55°C. The residual oil is diluted with ether, and the ether solution washed with water. The ether layer is dried over magnesium sulfate, filtered, and is then evaporated to yield 131.3 g of title product.

An nmr is run on the product to determine if any acetal which may have formed during the reaction (multiplet at 3.4) is present. If acetal is found in the product, 10% hydrochloric acid (85 ml) is added with stirring followed by the addition of sufficient amount of tetrahydrofuran to obtain a homogeneous solution. The solution is heated at 40—50°C for one hour. Water and ether are added, and the aqueous layer extracted with ether. The ether layers are combined, dried over magnesium sulfate and evaporated *in vacuo* to afford the title product.

## Example 2

Preparation of (*Z*)-*cis* and *trans*-3-($\beta$-cyanostyryl)-2,2-dimethyl-cyclopropanecarboxylic acid

Phenylacetonitrile is added to a stirred solution of sodium ethoxide in absolute ethanol (prepared from 1.1 g sodium and 100 ml of ethanol). Next, a solution of 3-formyl-2,2-dimethyl-cyclopropanecarboxylic acid ethyl ester (8.5 g) in absolute ethanol (15 ml) is added over 5 minutes. The reaction mixture is stirred for 12 hours at room temperature and then heated on a steam bath for 0.5 hour. The alcohol is removed *in vacuo*, and the residue washed with water (250 ml) and ether (150 ml). The

aqueous layer is acidified with concentrated hydrochloric acid. The resulting cream colored slurry is filtered and the isolated product dried. The product is dissolved in ether and precipitated with hexane to afford 6.1 g of white crystals, m.p. 186—188°C.

By the above procedure, a number of cyclopropanecarboxylic acids are prepared. These are listed in Table I below, together with their melting points.

TABLE I

Substituted Cyclopropanecarboxylic Acids

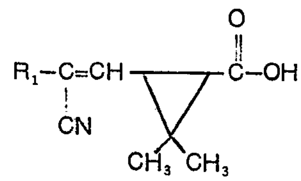

| $R_1$ | m.p. °C. |
|---|---|
| 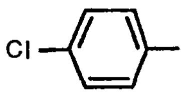 | 190–208 |
| 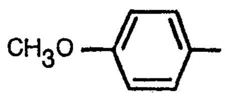 | 188–198 |
| 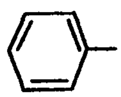 | 120–142 |
| 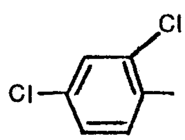 | 130–145 |
| 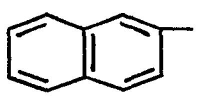 | 217–218 |
| 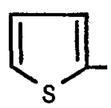 | 140–143 |
| 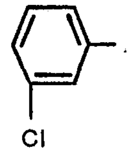 | 125–155 |
| 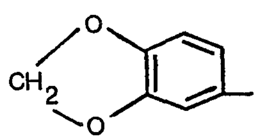 | 160–180 |

TABLE I (Continued)

| $R_1$ | m.p. °C. |
|---|---|
| 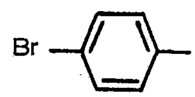 | 179–195 |
| 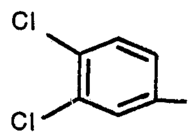 | 195–202 |
| 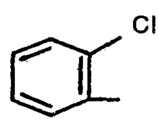 | * |
| 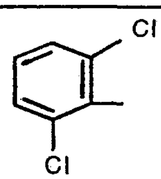 | 103–112 |
| 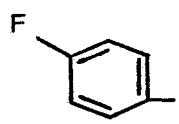 | 150–164 |
| 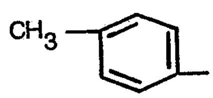 | 164–176 |
| 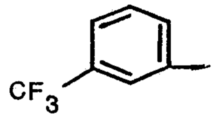 | 117–123 |

* = Gum – NMR TMS – $CDCl_3$
doublet – 6.30δ doublet 7.05δ

Example 3

Preparation of (*Z*)-*trans*-3-(β-cyanostyryl)-2,2-dimethyl-cyclopropanecarboxylic acid *m*-phenoxybenzyl ester

A mixture of (*Z*)-*cis* and *trans*-3-(β-cyanostyryl)-2,2-dimethyl-cyclopropanecarboxylic acid (3.5 g), benzene, (50 ml), and thionyl chloride (3.4 g) is refluxed for 1 hour. The benzene and excess thionyl chloride are then removed *in vacuo*. The residue is dissolved in benzene and added to a solution of *m*-phenoxybenzyl alcohol (2.9 g) in a mixture of benzene (50 ml) and pyridine (1.1 g). The reaction mixture is stirred at room temperature for 3 hours, and then filtered. The filtrate is evaporated *in vacuo*. The

residue is chromatographed on a silica gel column, and eluted with ethyl acetate and hexane to afford the title product.

Analysis calculated for $C_{28}H_{25}O_3N$: C 79.41; H 5.95; N 3.31;
Found: C 78.58; H 6.41; N 2.84.

By the above procedure, a number of cyclopropanecarboxylic acid esters are prepared. These are listed in Table II below, together with the corresponding analytical data.

TABLE II

Cyclopropanecarboxylic acid esters

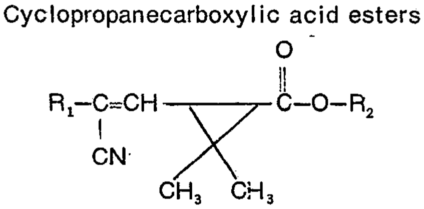

| $R_1$ | $R_2$ | Analysis: Calculated | Analysis: Found |
|---|---|---|---|
| 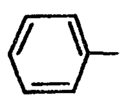 | CMPB | C 77.66<br>H 5.39<br>N 6.24 | C 76.65<br>H 5.71<br>N 5.88 |
| 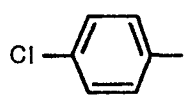 | MPB | C 73.43<br>H 5.28<br>N 3.06 | C 73.35<br>H 5.44<br>N 2.89 |
| 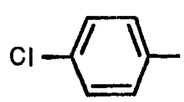 | CMPB | C 72.12<br>H 4.80<br>N 5.80 | C 71.44<br>H 5.25<br>N 5.03 |
| 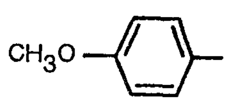 | MPB | C 76.80<br>H 6.00<br>N 3.09 | C 76.02<br>H 5.89<br>N 2.61 |
| 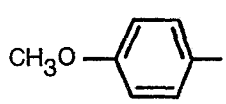 | CMPB | C 75.29<br>H 5.48<br>N 5.86 | C 74.93<br>H 5.35<br>N 5.68 |
| 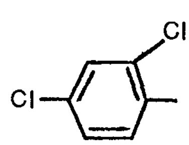 | MPB | C 68.30<br>H 4.71<br>N 2.85 | C 68.25<br>H 4.72<br>N 2.55 |
| 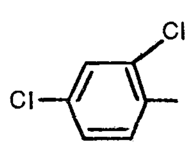 | CMPB | C 67.32<br>H 4.29<br>N 4.90 | C 66.64<br>H 4.46<br>N 4.90 |

TABLE II (Continued)

Cyclopropanecarboxylic acid esters

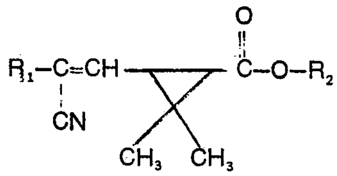

| $R_1$ | $R_2$ | Analysis | |
|---|---|---|---|
| | | Calculated | Found |
| 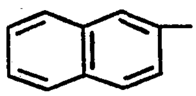 | CMPB | C 79.50<br>H 5.26<br>N 5.62 | C 79.20<br>H 5.69<br>N 5.54 |
| 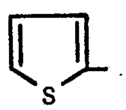 | CMPB | – | – |
| 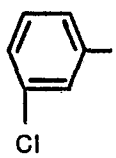 | MPB | C 73.58<br>H 5.25<br>N 3.04 | C 72.34<br>H 5.65<br>N 2.86 |
| 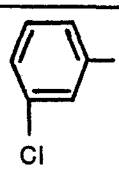 | CMPB | C 72.12<br>H 4.80<br>N 5.80 | C 71.47<br>H 5.10<br>N 5.42 |
| 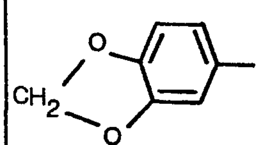 | MPB | C 74.50<br>H 5.39<br>N 3.00 | C 73.25<br>H 5.48<br>N 2.85 |
| 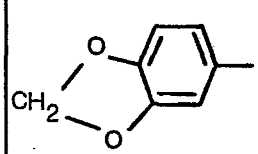 | CMPB | C 73.16<br>H 4.91<br>N 5.69 | C 71.59<br>H 5.22<br>N 5.33 |
| 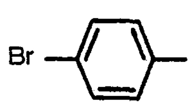 | MPB | C 66.93<br>H 4.81<br>N 2.79 | C 66.33<br>H 4.97<br>N 2.64 |

TABLE II (Continued)

Cyclopropanecarboxylic acid esters

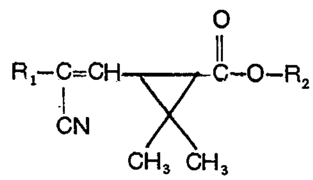

| $R_1$ | $R_2$ | Analysis | |
|---|---|---|---|
| | | Calculated | Found |
| 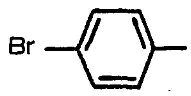 | CMPB | C 66.04<br>N 4.40<br>N 5.31 | C 65.58<br>H 4.74<br>N 4.95 |
| 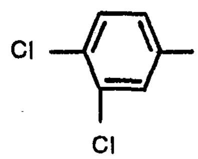 | MPB | C 68.30<br>H 4.71<br>N 2.85 | C 68.60<br>H 4.84<br>N 2.58 |
| 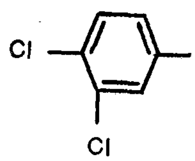 | CMPB | C 67.32<br>H 4.29<br>N 5.42 | C 66.55<br>H 4.64<br>N 5.13 |
| 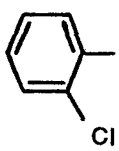 | MPB | C 73.44<br>H 5.28<br>N 3.06 | C 73.12<br>H 5.25<br>N 2.71 |
| 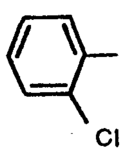 | CMPB | C 72.12<br>H 4.80<br>N 5.80 | C 70.85<br>H 5.03<br>N 5.22 |
| 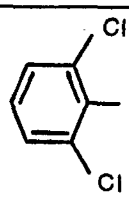 | MPB | C 68.30<br>H 4.71<br>N 2.85 | C 67.83<br>H 4.98<br>N 2.59 |
| 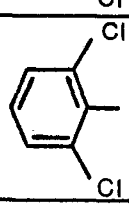 | CMPB | C 67.32<br>H 4.29<br>N 5.41 | C 65.13<br>H 4.11<br>N 5.14 |

TABLE II (Continued)

Cyclopropanecarboxylic acid esters

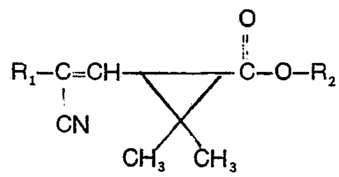

| $R_1$ | $R_2$ | Analysis |  |
|---|---|---|---|
| | | Calculated | Found |
| 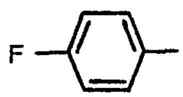 *trans* | MPB | C 76.17<br>H 5.48<br>N 2.86 | C 75.29<br>H 5.63<br>N 2.86 |
| 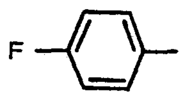 *cis* | MPB | C 76.17<br>H 5.48<br>N 2.86 | C 76.52<br>H 5.61<br>N 2.69 |
| 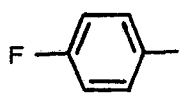 | CMPB | C 74.67<br>H 4.97<br>N 6.00 | C 74.64<br>H 5.15<br>N 5.90 |
| 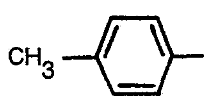 *trans* | MPB | C 79.61<br>H 6.22<br>N 3.20 | C 79.39<br>H 6.44<br>N 3.01 |
| 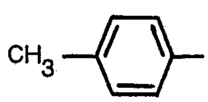 *cis* | MPB | C 79.61<br>H 6.22<br>N 3.20 | C 79.33<br>H 6.22<br>N 2.85 |
| 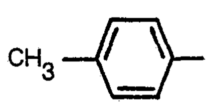 | CMPB | C 77.90<br>H 5.67<br>N 6.06 | C 74.54<br>H 5.36<br>N 5.63 |
| 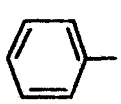 | 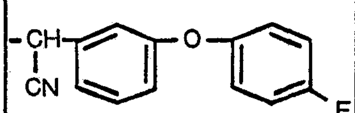 | C 74.67<br>H 4.97<br>N 6.00 | C 74.32<br>H 5.11<br>N 5.55 |

TABLE II (Continued)

Cyclopropanecarboxylic acid esters

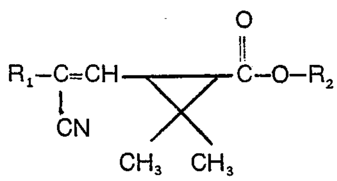

| $R_1$ | $R_2$ | Analysis | |
|---|---|---|---|
| | | Calculated | Found |
| 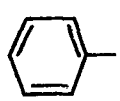 | 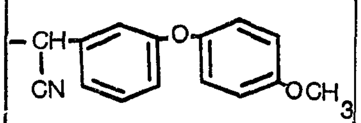 | C 75.30<br>H 5.48<br>N 5.85 | C 74.02<br>H 5.49<br>N 5.84 |
| 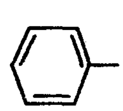 | 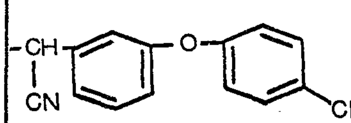 | C 72.11<br>H 4.80<br>N 5.80 | C 72.43<br>H 4.97<br>N 5.52 |

MPB = 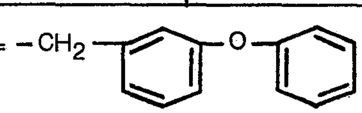 CMPB = 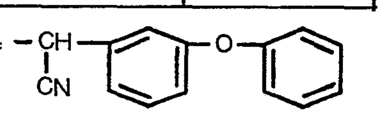

Example 4

Preparation of 2-Formyl-3-methylcyclopropanecarboxylic acid ethyl ester

Crotonaldehyde (14.0 g; 0.2 mole) is added dropwise to a gently refluxing solution of tetrahydrothiophene ylid (34.0 g; 0.2 mole) in dry acetone (200 ml). Heating at reflux is continued for about 15 minutes after the addition is completed, then the reaction mixture is concentrated on a rotary evaporator at 50°C. Distillation of this concentrate at reduced pressure affords 15.32 g (49.1%) of title product, a pale yellow oil, b.p. 0.1 mm, 44—45°C. The structure is confirmed by IR and NMR.

Example 5

Preparation of (*Z*)-*cis* and *trans*-2-($\beta$-cyanostyryl)-3-methylcyclopropanecarboxylic acid

A solution of sodium ethoxide is prepared by adding sodium spheres (1.15 g, 0.05 mole) to absolute ethanol (100 ml) with stirring until a solution is obtained. Phenylacetonitrile (5.86 g, 0.05 mole) and 2-formyl-3-methylcyclopropanecarboxylic acid methyl ester (7.81 g, 0.05 mole) are added to the above solution at room temperature. The resultant clear solution is stirred for 18 hours at room temperature, then treated at reflux for about 0.5 hours. The reaction mixture is then cooled, evaporated *in vacuo*, and the residue dissolved in water. The aqueous solution is extracted (3×) with ether and is then acidified with concentrated hydrochloric acid. A brown solid precipitates and is isolated by filtration and dried to afford 8.78 g (77.3%) of a tacky brown solid. NMR data supports the presence of both isomers (the olefin proton of the two isomers appearing as doublets at 6.17 ppm and 6.96 ppm).

Example 6

Preparation of (*Z*)-*cis* and *trans*-2-($\beta$-cyanostyryl)-3-methylcyclopropanecarboxylic acid, $\alpha$-cyano-*m*-phenoxybenzyl ester

By the procedure of Example 3, (*Z*)-*cis* and *trans*-2-($\beta$-cyanostyryl)-3-methylcyclopropanecarboxylic acid (5.0 g, 0.022 mole) is reacted with thionyl chloride (d = 1.63; 1.75 ml), 0.24 mole), and when formation of the acid chloride is complete, it (the acid chloride) is further reacted with a mixture of $\alpha$-cyano-*m*-phenoxybenzyl alcohol (4.5 g, 0.02 mole) and pyridine (1.74 g, 0.022 mole) to yield a crude, gummy product. The crude is purified by dry column chromatography tography silica gel; 1:1 methylene chloride (hexane) to give 4.8 g (57.1%) of a yellow gum.

Analysis calculated for $C_{28}H_{22}N_2O_3$: C 77.40; H 5.10; N 6.45;
Found: C 76.30; H 5.23; N 6.24.

Example 7

Preparation of (*Z*)-*cis* and *trans*-3-(β-cyano-p-methylstyryl)-2,2-dimethyl-cyclopropanecarboxylic acid, *m*-phenoxybenzyl ester

*m*-Phenoxybenzylbromide (3.68 g; 0.014 mole) is added to a solution of 3-(β-cyano-*p*-methylstyryl)-2,2-dimethylcyclopropanecarboxylic acid (3.19 g; 0.0125 mole) and triethylamine (1.27 g; 0.0125 mole) in dry dimethylformamide and the reaction mixture stirred for 18 hours at room temperature. The reaction mixture, containing some precipitated salts, is poured into water and extracted with ether (2 × 75 ml). The combined ether extracts are washed several times with water, then with sodium bicarbonate solution and saturated sodium chloride solution. Evaporation of the ether solution yields 3.42 g (62.5%) of a yellow oil. Purification of this yellow oil by dry column chromatography (silica gel; eluent 1:1 methylene chloride:hexane) affords 0.96 g of the *trans* isomer and 0.6 g of the *cis* isomer.

| | | | |
|---|---|---|---|
| Analysis calculated for $C_{29}H_{27}NO_3$: | C 79.61; | H 6.22; | N 3.20; |
| (*trans*) Found: | C 79.39; | H 6.44; | N 3.01; |
| (*cis*) Found: | C 79.33; | H 6.53; | N 2.85. |

Example 8

Preparation of (*Z*)-*cis* and *trans*-3-(β-cyanostyryl)-2,2-dimethyl-cyclopropanecarboxylic acid, α-cyano-*m*-(p-chlorophenoxy)benzyl ester by a phase transfer catalyst esterification process

Thionyl chloride (1.61 ml, 2.6 g; 0.022 mole), 3-(β-cyanostyryl)-2,2-dimethyl-cyclopropanecarboxylic acid (4.82 g; 0.02 mole) and toluene are mixed and heated at 100°C for 1 hour. The reaction mixture is cooled to 25°C and a solution of *m*-(*p*-chlorophenoxy)benzaldehyde (4.65 g; 0.02 mole) and 18-crown-6 (0.49 g; 0.002 mole) in toluene (10 ml) is added. To this reaction mixture a solution of potassium cyanide (2.6 g; 0.04 mole) in water (6 ml) is added slowly while maintaining the temperature between 20—25°C. The reaction mixture is then stirred for 18 hours at room temperature. The resultant black reaction mixture is poured in water and extracted with ether (3×). The combined ether extracts are washed with water, 10% hydrochloric acid, 10% sodium bicarbonate, water and saturated sodium chloride solution. The ether layer is dried over sodium sulfate and evaporated *in vacuo* to leave a brown gum. This gum is purified by dry column chromatography (silica gel; eluent: 1:1 methylene chloride:hexane) to afford the title product, a yellow glass (1.0 g, 8.6%).

| | | | |
|---|---|---|---|
| Analysis calculated for $C_{29}H_{23}ClN_2O_3$: | C 72.12; | H 4.80; | N 5.80; |
| Found: | C 72.43; | H 4.97; | N 5.53. |

Example 9

Preparation of 20% w/w emulsifiable concentrates containing cyanovinyl pyrethroids of the invention

Toxicants

(1). (*Z*)-*trans*-3-(*p*-chloro-β-cyanostyryl)-2,2-dimethyl-cyclopropanecarboxylic acid, α-cyano-*m*-phenoxybenzyl ester.

(2). (*Z*)-*trans*-3-(β-cyanostyryl)-2,2-dimethylcyclopropanecarboxylic acid, α-cyano-*m*-phenoxybenzyl ester.

(3). (*Z*)-*trans*-3-(2,4-dichloro-β-cyanostyryl)-2,2-dimethylcyclopropanecarboxylic acid, α-cyano-*m*-phenoxybenzyl ester.

Concentrates

| Component | wt. in g. |
|---|---|
| Toxicant | 15.0 |
| Atlox® 3403F/3404F; 50:50 mixture[(a)] | 10.125 |
| xylene | 6.75 |
| "HAN"[(b)] | 35.625 |
| Total: | 67.500 |

(a) Atlox® 3403F, a polyoxyethylene alkyl aryl ether-alkyl aryl sulfonate blend; nonionic surfactant; specific gravity: 1.02 at 20°C; wt in kg/l = 1.018 (8.5 lb/gal); approximate viscosity at 25°C = 130 cps; flash point (C.O.C.; °F) = 100;

Atlox® 3403F, a polyoxyethylene ether polyoxyethylene glyceride-alkyl aryl sulfonate blend; anionic surfactant; specific gravity 1.05 at 25°C; wt in kg/l = 1.052 (8.7 lb/gal); approximate viscosity

at 25°C = 3700 cps; flash point (C.O.C.; °F) = 160. Both are proprietary products of ICI United States Inc. Atlas Chemicals Division, Wilmington, Delaware.
(b) "HAN" = Heavy Aromatic Naphtha, boiling range 165°C to 282°C; Mixed aniline point °C = 28.0; freeze point °C = 36; aromatic content: 80%.

Method of preparation

The toxicant is preheated to about >85°C to obtain a flowable melt, and then mixed with the surfactants, xylene and "HAN". The resultant mixture is heated and stirred to obtain a clear solution.

Standard emulsification test (50λ/33 ml medium hard water) indicates, that although on addition the emulsifiable concentrate tends to float on top of the water layer, with agitation (or shaking) good emulsions are obtained, which remain quite stable with very little creaming on top.

The average micelle sizes of the aqueous emulsions prepared from the above concentrates are determined using a Coulter Counter with the following results:

| Emulsion containing Toxicant No. | Micelle size in microns |
|---|---|
| 1 | 4.5 |
| 2 | 2.2 |
| 3 | 2.4 |

Example 10

Evaluation of the efficacy of the compounds of the invention for the control of *Boophilus microplus* larvae

Effective control of acarina larvae is demonstrated in the following tests with larvae of *Boophilus microplus*, a one-host tick which can remain on a single host through its three life stages, i.e. larva, nymph and adult. In these tests a 10% acetone/90% water mixture contains the test compound at the concentrations indicated in Table III below. Twenty larvae are enclosed in a pipet sealed at one end with a gauze material, and a solution, containing the test compound at the concentrations given, is then drawn through the pipet with a vacuum hose simulating a spray system. The ticks are then held for 48 hours at room temperature, and percent mortality rates are then determined. The results obtained with the various compounds are tabulated in Table III below.

TABLE III

Efficacy of cyanovinyl pyrethroids for the control of
*Boophilus microplus* larvae

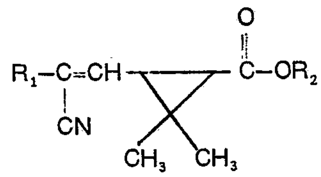

| $R_1$ | $R_2$ | Concentration of Toxicant in spray solution (ppm) | Percent Mortality of *Boophilus microplus* larvae |
|---|---|---|---|
| 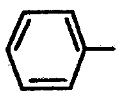 | CMPB | 0.001 | 100 |
| 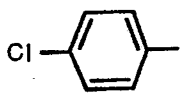 | MPB | 0.001 | 100 |
| 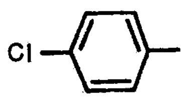 | CMPB | 0.001 | 100 |
| 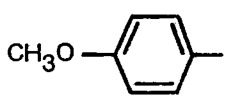 | MPB | 1.0 | 100 |
| 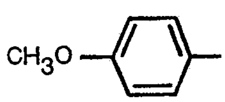 | CMPB | 1.0 | 100 |
| 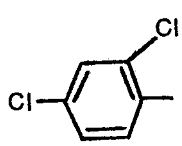 | MPB | 0.001 | 100 |
| 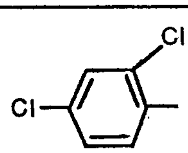 | CMPB | 0.001 | 100 |

TABLE III (Continued)

Efficacy of cyanovinyl pyrethroids for the control of
*Boophilus microplus* larvae

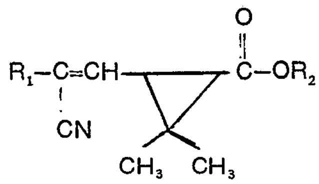

| $R_1$ | $R_2$ | Concentration of Toxicant in spray solution (ppm) | Percent Mortality of *Boophilus microplus* larvae |
|---|---|---|---|
| 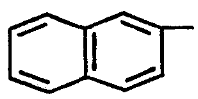 | CMPB | 100.0 | 100 |

MPB = 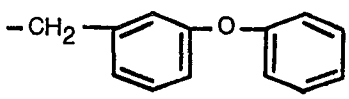

CMPB = 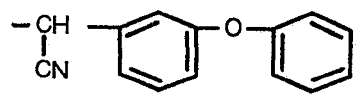

TABLE III (Continued)

Efficacy of cyanovinyl pyrethroids for the control of
*Boophilus microplus* larvae

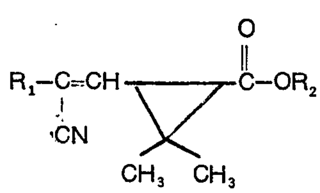

| $R_1$ | $R_2$ | Concentration of Toxicant in spray solution (ppm) | Percent Mortality of *Boophilus microplus* larvae |
|---|---|---|---|
| 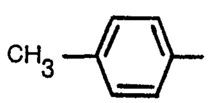 *(Z)-cis* | MPB | 100 | 100 |

TABLE III (Continued)

Efficacy of cyanovinyl pyrethroids for the control of
*Boophilus microplus* larvae

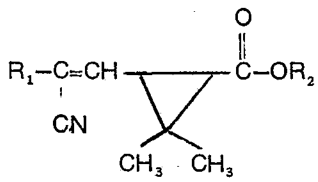

| $R_1$ | $R_2$ | Concentration of Toxicant in spray solution (ppm) | Percent Mortality of *Boophilus microplus* larvae |
|---|---|---|---|
| 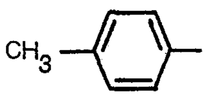 *(Z)-trans* | MPB | 100 | 100 |
| 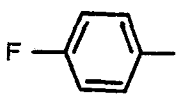 *(Z)-cis* | MPB | 100 | 100 |
| 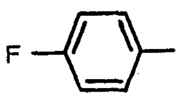 *(Z)-trans* | MPB | 100 | 30 |
| 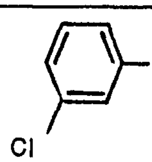 *(Z)-cis* | MPB | 100 | 100 |
| 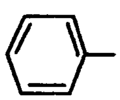 *(Z)-trans* | MPB | 100 | 0 |
| 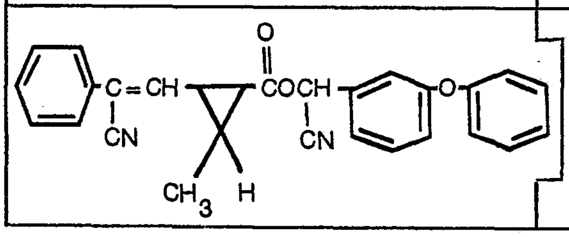 | | 1.0<br>0.1 | 100<br>50 |

Example 11

Suppression of fecundity and chemosterilant effect in Ixodidae

The efficacy of the compounds of the invention for suppression of fecundity and chemosterilant effect in ticks is demonstrated in the following tests wherein engorged adult female *Boophilus microplus*, multiple resistant strain, ticks which have dropped from cattle are collected and used for testing.

The compound to be tested is dissolved in a 35% acetone/65% water mixture in amounts sufficient to provide the concentrations indicated in Table IV below. Ten ticks per treatment are used and they are immersed in the test solution for three to 5 minutes, then removed and held in incubators for two to three weeks at 28°C. Counts of ticks laying eggs are then made and recorded. Eggs which were laid are placed in containers and kept for one month to observe hatching and to determine chemosterilant effect. Results of these tests are given in Table IV below.

TABLE IV

Efficacy of cyanovinyl pyrethroids expressed as % reduction of viable egg masses

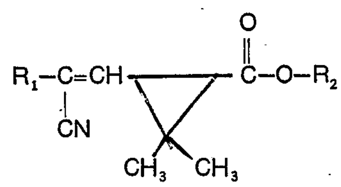

| $R_1$ | $R_2$ | Concentration in solution (ppm) | Percent reduction of viable egg masses |
|---|---|---|---|
| 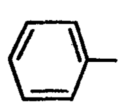 | CMPB | 31.2<br>16.0<br>8.0<br>8.0<br>4.0 | 95.9–99<br>85<br>65<br>84–94<br>62–70 |
| 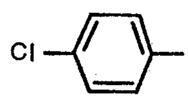 | MPB | 15<br>8<br>4 | 99.4<br>72.9–78<br>24.5–32 |
| 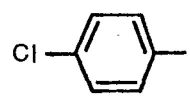 | CMPB | 4<br>2 | 99.4<br>97 |
| 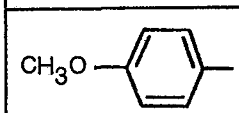 | MPB | 250<br>125<br>62<br>31 | 85.5–87.8<br>79.1–91.2<br>90.9–96.6<br>63.6 |
| 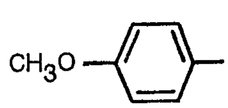 | CMPB | 31<br>15 | 91.8<br>73.5 |
| 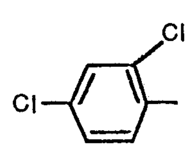 | MPB | 32<br>16<br>8 | 85.6–91<br>87.5–95<br>40–64 |

TABLE IV (Continued)

Efficacy of cyanovinyl pyrethroids expressed as % reduction of viable egg masses

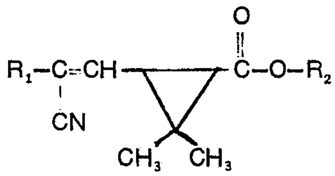

| $R_1$ | $R_2$ | Concentration in solution (ppm) | Percent reduction of viable egg masses |
|---|---|---|---|
| 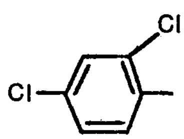 | CMPB | 4<br>2 | 97<br>55 |
| 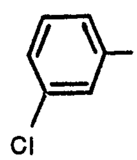 | CMPB | 31 | 98.8 |
| 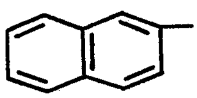 | CMPB | 15 | 92 |
| 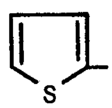 | CMPB | 100 | 100 |

MPB = 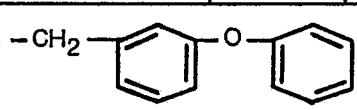 CMPB = 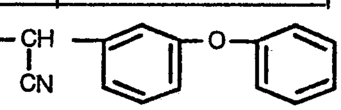

Example 12

Evaluation of the efficacy of cyanovinyl pyrethroids for the control of *Boophilus microplus* on cattle

Test Compounds

The 20% w/w emulsifiable concentrates of Example 6 are used in this experiment. Spray emulsions are prepared just prior to use from the above concentrates by diluting 11.25 ml and 22.5 ml of each with 75 liter of water to yield spray emulsions containing 30 and 60 ppm of active ingredient, respectively.

Test Animals

Holstein calves, 6 to 12 months old are experimentally infested with about 5000 larval ticks of a multiple resistant (resistant to organophosphate acaricides as well as to toxaphene and lindane) strain placed on each animal three times a week, starting 35 days pre-treatment. Three pre-treatment tick counts are made in days −6, −5 and −4. Calves are allocated to groups of five on the basis of these counts. One group serves as infected nontreated controls and one group each is used to evaluate the

test compounds at 30 and 60 ppm level of toxicant, respectively. On treatment day, each animal is sprayed with a total of 15 liters of emulsion delivered at 21.09 kg/cm² pressure with a "Teejet 8002 Excelsior" nozzle providing a fanshaped spray. Mortality counts (female ticks 4.5—8.0 mm) are taken over days 2 to 21 posttreatment for a total of 9 times. Residual effect is measured by six mortality counts taken over days 23 to 35 posttreatment. The results obtained, are summarised and tabulated in Table V below.

TABLE V

Evaluation of the efficacy of the compounds of the present invention for the control of multiple resistant ticks on cattle

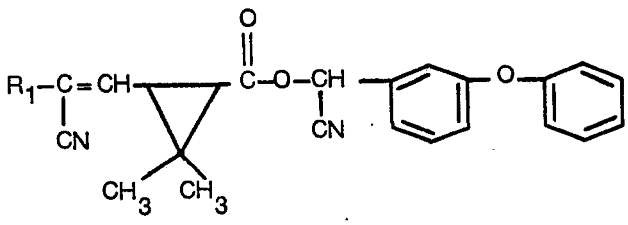

| $R_1$ | Toxicant Concentration (ppm) | Percent Mortality (post-treatment) | |
|---|---|---|---|
| | | Days 2 to 21 | Days 23 to 35 |
| 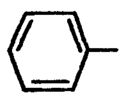 | 30<br>60 | 95.8<br>99.2 | 97.8<br>99.4 |
| 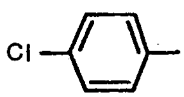 | 30<br>60 | 92.9<br>96.8 | 99.2<br>99.2 |
| 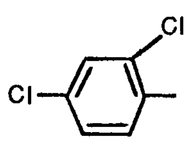 | 30<br>60 | 65<br>85.9 | 89.2<br>95.5 |

## Example 13

Evaluation of the Ixodicidal activity of cyanovinyl Pyrethroids against *Amblyomma hebreum*

*The ixodicidal activity of the compounds of the invention is demonstrated in the following tests* wherein juvenile adult *Amblyomma hebreum* ticks are collected and used for testing.

The compound to be tested is dissolved in a 35% acetone/65% water mixture in amounts sufficient to provide the concentrations indicated in Table VI below. Ten ticks per treatment (ten males or ten females) are used and they are immersed in the test solution for three to 5 minutes, then removed and held in incubators at 28°C. Percent mortality is determined at 24 and 96 hours post-immersion. The data obtained is tabulated in Table VI below.

TABLE VI

Percent Mortality of *Amblyomma hebreum* at 24 hours post-immersion

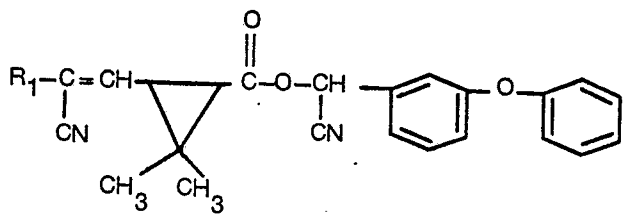

| $R_1$ | Concentration of Toxicant (ppm) | % Mortality at 24 hours | Remarks |
|---|---|---|---|
| 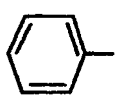 | 240<br>120<br>60<br>30<br>15<br>7.5<br>3.25 | 100<br>100<br>90<br>45<br>6<br>15<br>0 | <br><br>100% at 96 hours<br>75% at 96 hours |
| 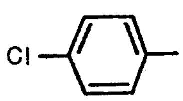 | 240<br>120<br>60<br>30<br>15<br>7.5<br>3.25 | 95<br>70<br>45<br>75<br>15<br>0<br>0 | |
| 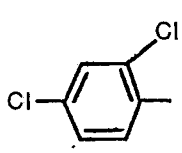 | 240<br>120<br>60<br>30<br>15<br>7.5<br>3.25 | 100<br>100<br>65<br>60<br>50<br>0<br>0 | |

**Claims for the Contracting States: BE CH DE FR GB IT NL SE**

1. A method for the control of ticks comprising contacting the insects or ectoparasites, or applying to their hosts or to their habitat a compound of the formula:

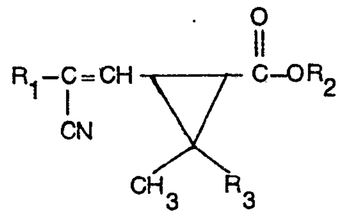

–(I)

wherein $R_1$ is 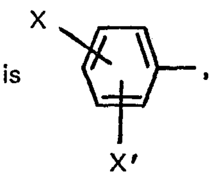, 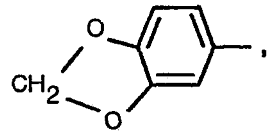, 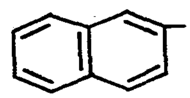 or 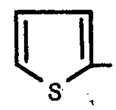 ;

$R_2$ is a moiety 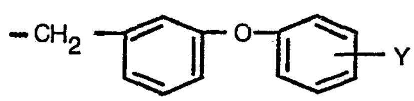 or 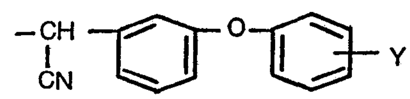

$R_3$ is hydrogen or methyl; X and X′ are each hydrogen, halogen, $C_1$—$C_3$ alkyl, $C_1$—$C_3$ alkoxy or haloalkyl $C_1$—$C_2$; Y is hydrogen, halogen, methyl or methoxy in amounts sufficient to achieve control of the ticks.

2. A method according to Claim 1, wherein $R_1$ is

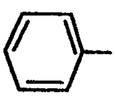, 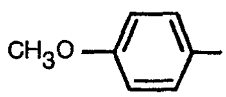, 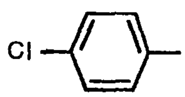, 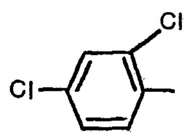, 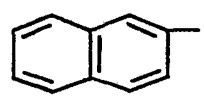 or 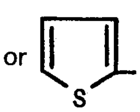 ;

$R_2$ is 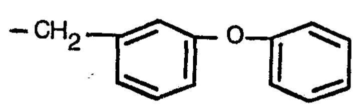 or 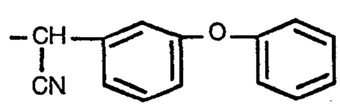;

$R_3$ is methyl.

3. A method according to Claim 2, wherein the compound is (*Z*) - *trans* - 3 - (*β* - cyanostyryl) - 2,2 - dimethylcyclopropanecarboxylic acid, *α* - cyano - *m* - phenoxybenzyl ester; (*Z*) - *trans* - 3 - (*p* - chloro - *β* - cyanostyryl) - 2,2 - dimethylcyclopropanecarboxylic acid, *m* - phenoxybenzyl ester; (*Z*) - *trans* - 3 - (*p* - chloro - *β* - cyanostyryl) - 2,2 - dimethylcyclopropanecarboxylic acid, *α* - cyano - *m* - phenoxybenzyl ester; (*Z*) - *trans* - 3 - (*β* - cyano - *p* - methoxystyryl) - 2,2 - dimethylcyclopropanecar - boxylic acid, *m* - phenoxybenzyl ester; (*Z*) - *trans* - 3 - (*β* - cyano - p - methoxystyryl) - 2,2 - dimethyl - cyclopropanecarboxylic acid, *α* - cyano - *m* - phenoxybenzyl ester; (*Z*) - *trans* - 3 - (2,4 - dichloro - *β* - cyanostyryl) - 2,2 - dimethylcyclopropanecarboxylic acid, *m* - phenoxybenzyl ester; or (*Z*) - *trans* - 3 - (2,4 - dichloro - *β* - cyanostyryl) - 2,2 - dimethylcyclopropanecarboxylic acid, *α* - cyano - *m* - phenoxy - benzyl ester.

4. A method according to any preceding claim, wherein the compound is applied at the rate of 0.001 ppm to 100 ppm.

5. A compound of the formula:

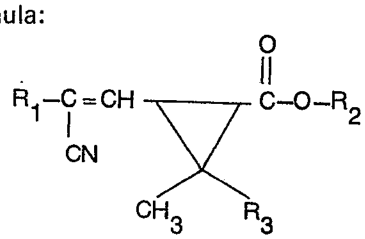

wherein $R_1$ is 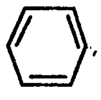, 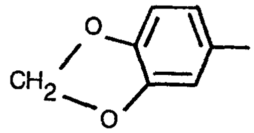, 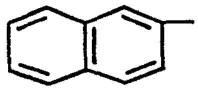 or 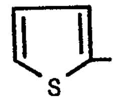 ;

$R_2$ is 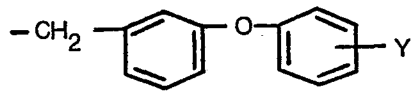 or 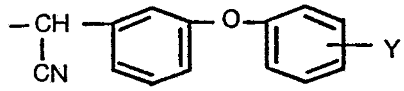

$R_3$ is hydrogen or methyl; and Y is hydrogen, halogen, methyl or methoxy.

6. A compound according to Claim 5,

wherein $R_1$ is 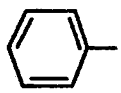, 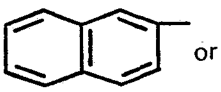 or 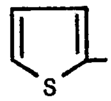;

$R_2$ is 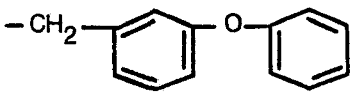 or 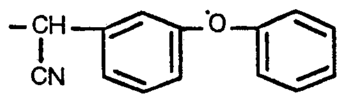;

and $R_3$ is methyl.

7. The compounds according to Claim 5; (*Z*) - *trans* - 3 - (*β* - cyanostyryl) - 2,2 - dimethyl - cyclopropanecarboxylic acid, *α* - cyano - *m* - phenoxybenzyl ester; (*Z*) - *trans* - 3 - (*β* - cyanostyryl) - 2,2 - dimethyl - cyclopropanecarboxylic acid, *m* - phenoxybenzyl ester; (*Z*) - *trans* - 3 - [2 - cyano - 2(2 - naphthyl)vinyl] - 2,2 - dimethyl - cyclopropanecarboxylic acid, *α* - cyano - *m* - phenoxybenzyl ester or (*E*) - *trans* - 3 - [2 - cyano - 2 - (2 - thienyl)vinyl] - 2,2 - dimethyl - cyclopropanecarboxylic acid, *α* - cyano - *m* - phenoxybenzyl ester.

8. A method for the preparation of a compound of the structure represented by Formula (I):

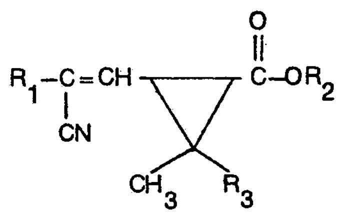

(I)

wherein $R_1$, $R_2$, and $R_3$, are as defined in claim 5, comprising condensing an aldehyde of formula (III):

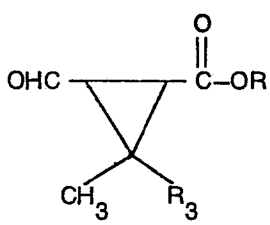

(III),

wherein R is ($C_1$—$C_3$) alkyl and $R_3$ is as hereinabove defined, with a compound having an active methylene group and represented by formula $R_1$—$CH_2$—CN wherein $R_1$ is as hereinabove defined in the presence of a base such as sodium or potassium ethoxide and a lower ($C_1$—$C_3$) alcohol to obtain a cyclopropanecarboxylic acid of formula (IV):

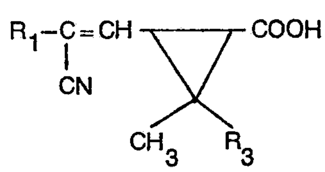

(IV)

converting the carboxylic acid (IV) to the corresponding acid chloride (V):

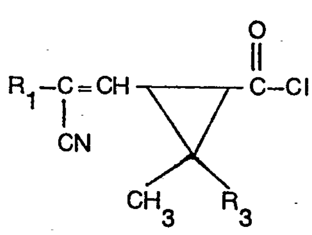

(V)

with thionyl chloride in the presence of an aromatic solvent of benzene, toluene or xylene, and reacting the acid chloride (V) with an alcohol of the formula

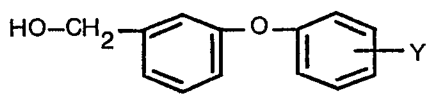 or 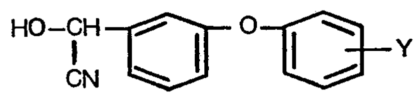

wherein Y is as hereinabove defined, in the presence of pyridine and an inert aromatic solvent selected from benzene, toluene and xylene to obtain the compound of formula (I).

9. A method according to Claim 8,

wherein $R_1$ is 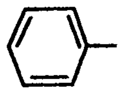 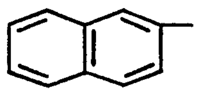 or 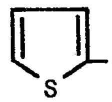 ;

$R_2$ is 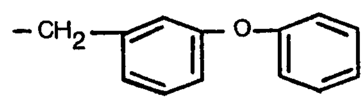 or 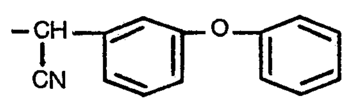 ;

and $R_3$ is methyl; or a *cis* or *trans* isomer thereof, or an isomeric mixture thereof.

10. A tickicide composition comprising a compound of the formula:

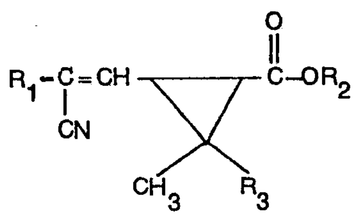

(I)

wherein $R_1$, $R_2$ and $R_3$ are as defined in Claim 1 or Claim 2 as an active ingredient, in combination with an inert agricultural adjuvant.

11. A tickicide composition according to Claim 10, wherein said active compound is as defined in Claim 3.

**Claims for the Contracting State: AT**

1. A method for the control of ticks comprising contacting the insects or ectoparasites, or applying to their hosts or to their habitat a compound of the formula:

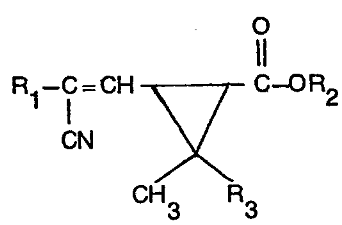

–(I)

wherein $R_1$ is 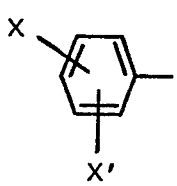 , 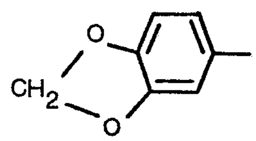 , 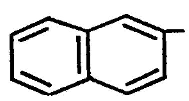 or 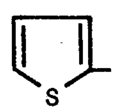 ;

$R_2$ is a moiety 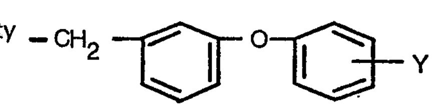 or 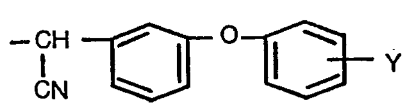

$R_3$ is hydrogen or methyl; X and X′ are each hydrogen, halogen, $C_1$—$C_3$ alkyl, $C_1$—$C_3$ alkoxy or haloalkyl $C_1$—$C_2$; Y is hydrogen, halogen, methyl or methoxy in amounts sufficient to achieve control of the ticks.

2. A method according to Claim 1, wherein $R_1$ is

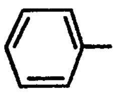, 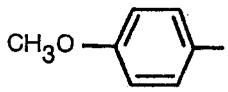, 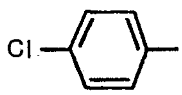, 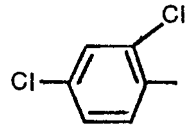, 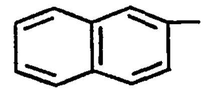 or 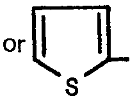;

$R_2$ is 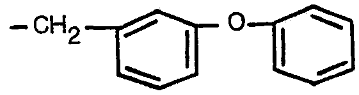 or 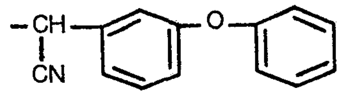;

$R_3$ is methyl.

3. A method according to Claim 2, wherein the compound is (*Z*) - *trans* - 3 - (β - cyanostyryl) - 2,2 - dimethylcyclopropanecarboxylic acid, α - cyano - *m* - phenoxybenzyl ester; (*Z*) - *trans* - 3 - (*p* - chloro - β - cyanostyryl) - 2,2 - dimethylcyclopropanecarboxylic acid, *m* - phenoxybenzyl ester; (*Z*) - *trans* - 3 - (*p* - chloro - β - cyanostyryl) - 2,2 - dimethylcyclopropanecarboxylic acid, α - cyano - *m* - phenoxybenzyl ester; (*Z*) - *trans* - 3 - (β - cyano - *p* - methoxystyryl) - 2,2 - dimethylcyclopropanecar - boxylic acid, *m* - phenoxybenzyl ester; (*Z*) - *trans* - 3 - (β - cyano - p - methoxystyryl) - 2,2 - dimethyl - cyclopropanecarboxylic acid, α - cyano - *m* - phenoxybenzyl ester; (*Z*) - *trans* - 3 - (2,4 - dichloro - β - cyanostyryl) - 2,2 - dimethylcyclopropanecarboxylic acid, *m* - phenoxybenzyl ester; or (*Z*) - *trans* - 3 - (2,4 - dichloro - β - cyanostyryl) - 2,2 - dimethylcyclopropanecarboxylic acid, α - cyano - *m* - phenoxy - benzyl ester.

4. A method according to any preceding claim, wherein the compound is applied at the rate of 0.001 ppm to 100 ppm.

5. A method for the preparation of a compound of the structure represented by formula (I):

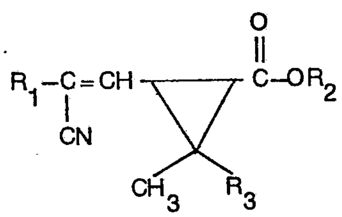

(I)

wherein $R_1$ is 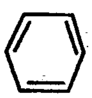 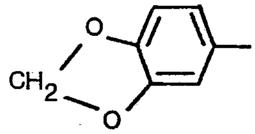, 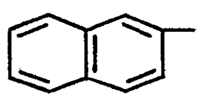 or 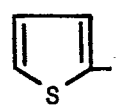;

$R_2$ is a moiety 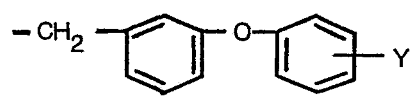 or 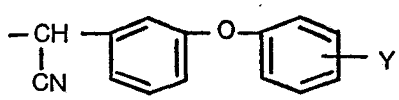;

$R_3$ is hydrogen or methyl; and Y is hydrogen halogen methyl or methoxy; comprising condensing an aldehyde of formula (III):

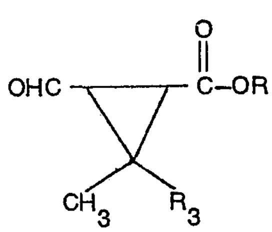

(III),

wherein R is ($C_1$—$C_3$) alkyl and $R_3$ is as hereinabove defined, with a compound having an active methylene group and represented by formula $R_1$—$CH_2$—CN wherein $R_1$ is as hereinabove defined in the presence of a base such as sodium or potassium ethoxide and a lower ($C_1$—$C_3$) alcohol to obtain a cyclopropanecarboxylic acid of formula (IV):

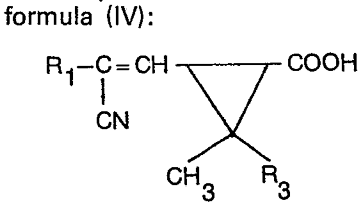

(IV)

converting the carboxylic acid (IV) to the corresponding acid chloride (V):

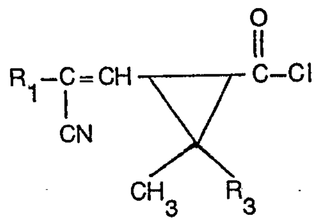

(V)

with thionyl chloride in the presence of an aromatic solvent of benzene, toluene or xylene, and reacting the acid chloride (V) with an alcohol of the formula

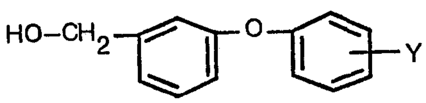 or 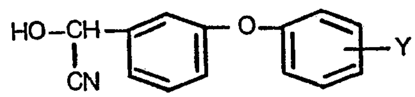

wherein Y is as hereinbefore defined, in the presence of pyridine and an inert aromatic solvent selected from benzene, toluene and xylene to obtain the compound of formula (I).

6. A method according to Claim 5, wherein $R_1$ is

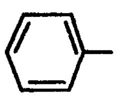, 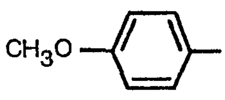, 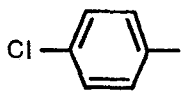, 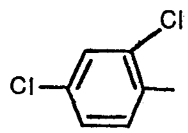, 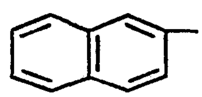 or 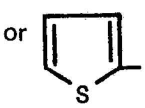;

$R_2$ is 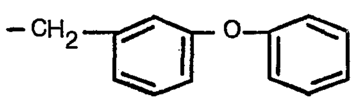 or 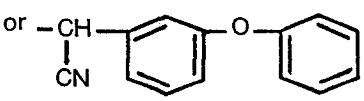;

and $R_3$ is methyl; or a *cis* or *trans* isomer thereof, or an isomeric mixture thereof.

7. A tickicide composition comprising a compound of the formula:

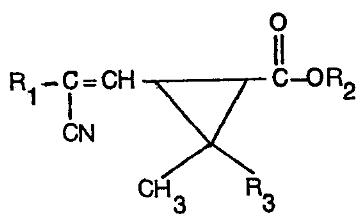

(I)

wherein $R_1$, $R_2$ and $R_3$ are as defined in Claim 1 or Claim 2 as an active ingredient, in combination with an inert agricultural adjuvant.

8. A tickicide composition according to Claim 7, wherein said active compound is as defined in Claim 3.

9. A method of preparing a tickicide composition, comprising incorporating a compound of the formula:

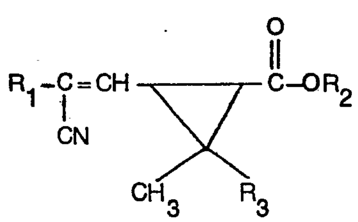

(I)

wherein $R_1$, $R_2$ and $R_3$ are as defined in Claim 1 or Claim 2, with an inert agricultural adjuvant.

**Patentansprüche für die Vertragsstaaten: BE CH DE FR GB IT NL SE**

1. Verfahren zur Bekämpfung von Zecken und/oder Milben, dadurch gekennzeichnet, daß man die Insekten oder Ektoparasiten oder ihre Wirtstiere oder ihren Lebensraum kontaktiert bzw. behandelt mit einer Verbindung der Formel:

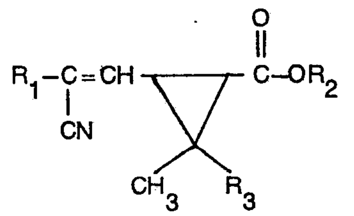 —(I)

wobei $R_1$ für 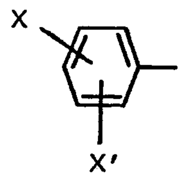, 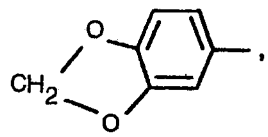, 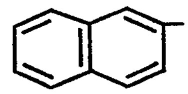 oder 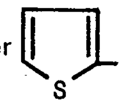;

steht; $R_2$ für eine Einheit 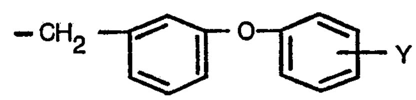 oder 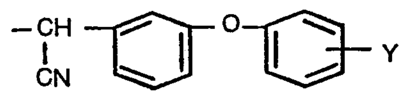

steht; $R_3$ für Wasserstoff oder Methyl steht; X und X′ jeweils Wasserstoff, Halogen, $C_1$—$C_3$-Alkyl, $C_1$—$C_3$-Alkoxy oder Halogenalkyl-$C_1$—$C_2$ bedeuten und Y für Wasserstoff, Halogen, Methyl oder Methoxy steht; und zwar in Mengen, die ausreichen, um ein Bekämpfung der Zecken und/oder Milben zu erreichen.

2. Verfahren nach Anspruch 1, dadurch gekennzeichnet, daß $R_1$ für

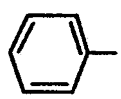, 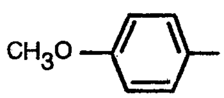, 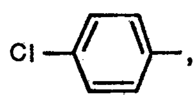, 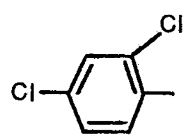, 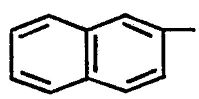 oder 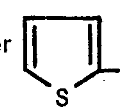;

$R_2$ für 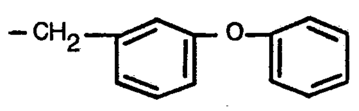 oder 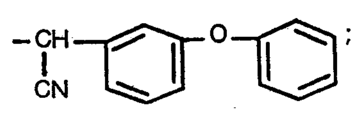;

steht; und $R_3$ für Methyl steht.

3. Verfahren nach Anspruch 2, dadurch gekennzeichnet, daß es sich bei der Verbindung handelt um (*Z*) - *trans* - 3 - (*β* - Cyanostyryl) - 2,2 - dimethylcyclopropancarbonsäure, *α* - Cyano - *m* - phenoxybenzylester; (*Z*) - *trans* - 3 - (*p* - Chlor - *β* - cyanostyryl) - 2,2 - dimethylcyclopropancarbonsäure, *m* - Phenoxybenzylester; (*Z*) - *trans* - 3 - (*p* - Chlor - *β* - cyanostyryl) - 2,2 - dimethylcyclopropancarbonsäure, *α* - Cyano - *m* - phenoxybenzylester; (*Z*) - *trans* - 3 - (*β* - Cyano - *p* - methoxystyryl) - 2,2 - dimethylcyclopropancarbonsäure, *m* - Phenoxybenzylester; (*Z*) - *trans* - 3 - (*β* - Cyano - p - methoxystyryl) - 2,2 - dimethylcyclopropancarbonsäure, *α* - Cyano - *m* - phenoxybenzylester; (*Z*) - *trans* - 3 - (2,4 - Dichlor - *β* - cyanostyryl) - 2,2 - dimethylcyclopropancarbonsäure, *m* -Phenoxybenzylester; oder (*Z*) - *trans* - 3 - (2,4 - Dichlor - *β* - cyanostyryl) - 2,2 - dimethylcyclopropancarbonsäure, *α* - Cyano - *m* - phenoxybenzylester.

4. Verfahren nach einem der vorstehenden Ansprüche, dadurch gekennzeichnet, daß die Verbindung mit einer Rate von 0,001 ppm bis 100 ppm appliziert wird.

5. Verbindung der Formel:

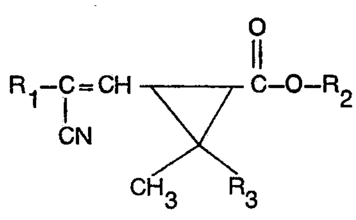

wobei $R_1$ für 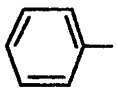, 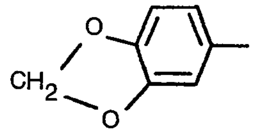, 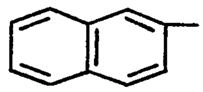 oder 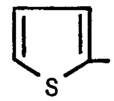 steht;

$R_2$ für 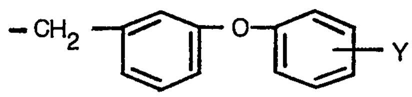 oder 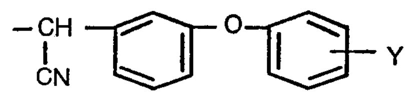

steht; $R_3$ für Wasserstoff oder Methyl steht und Y für Wasserstoff, Halogen, Methyl oder Methoxy steht.

6. Verbindung nach Anspruch 5, dadurch gekennzeichnet, daß $R_1$ für

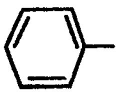, 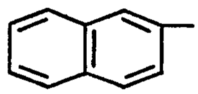 oder 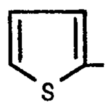 steht;

$R_2$ für 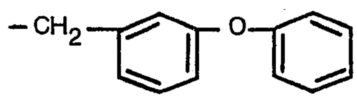 oder 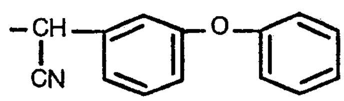

steht; und $R_3$ für Methyl steht.

7. Verbindungen nach Anspruch 5, nämlich (*Z*) - *trans* - 3 - ($\beta$ - Cyanostyryl) - 2,2 - dimethyl - cyclopropancarbonsäure, $\alpha$ - Cyano - *m* - phenoxybenzylester; (*Z*) - *trans* - 3 - ($\beta$ - Cyanostyryl) - 2,2 - dimethyl - cyclopropancarbonsäure, *m* - Phenoxybenzylester; (*Z*) - *trans* - 3 - [2 - Cyano - 2(2 - naphthyl)vinyl] - 2,2 - dimethyl - cyclopropancarbonsäure, $\alpha$ - Cyano - *m* - phenoxybenzylester oder (*E*) - *trans* - 3 - [2 - Cyano - 2 - (2 - thienyl)vinyl] - 2,2 - dimethyl - cyclopropancarbonsäure, $\alpha$ - Cyano - *m* - phenoxybenzylester.

8. Verfahren zur Herstellung einer Verbindung der Struktur gemäß Formel (I):

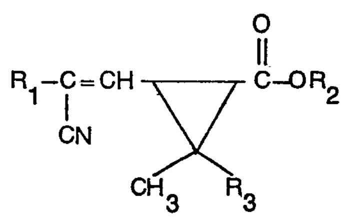

(I)

wobei $R_1$, $R_2$ und $R_3$ die in Anspruch 5 angegebene Bedeutung haben, dadurch gekennzeichnet, daß man einen Aldehyd der Formel (III):

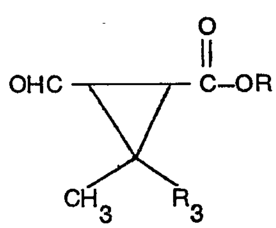

(III),

wobei R für $C_1$—$C_3$-Alkyl steht und $R_3$ die oben angegebene Bedeutung hat, mit einer Verbindung, welche eine aktive Methylengruppe aufweist und durch die Formel $R_1$—$CH_2$—CN dargestellt ist, wobei $R_1$ die oben angegebene Bedeutung hat, in Gegenwart einer Base wie Natrium- oder Kaliummethoxid und einem niederen $C_1$—$C_3$-Alkohol kondensiert, um eine Cyclopropancarbonsäure der Formel (IV) zu erhalten:

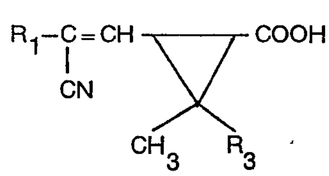

(IV)

die Carbonsäure (IV) in das korrespondierende Säurechlorid (V):

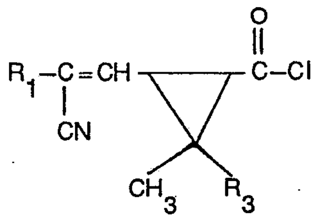

(V)

mit Thionylchlorid in Gegenwart eines aromatischen Lösungsmittels wie Benzol, Toluol oder Xylol umwandelt und das Säurechlorid (V) mit einem Alkohol der Formel:

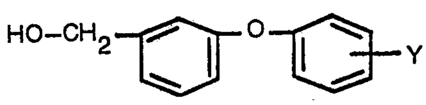

oder

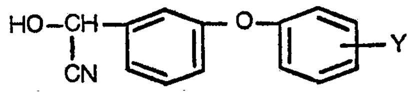

wobei Y die oben angegebene Bedeutung hat, in Gegenwart von Pyridin und einem inerten aromatischen Lösungsmittel, ausgewählt unter Benzol, Toluol und Xylol, umsetzt, um die Verbindung der Formel (I) zu erhalten.

9. Verfahren nach Anspruch 8, dadurch gekennzeichnet, daß $R_1$ für

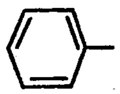

,

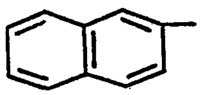

oder

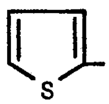

steht;

$R_2$ für

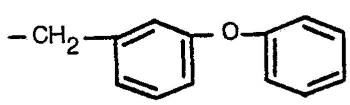

oder

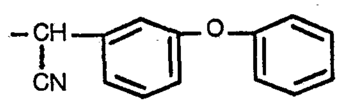

.

steht und $R_3$ für Methyl steht; oder ein *cis*- oder *trans*-Isomeres derselben oder ein Isomerengemisch derselben.

10. Schädlingsbekämpfungsmittel, gekennzeichnet durch eine Verbindung der Formel:

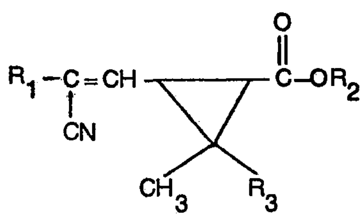

(I)

wobei $R_1$, $R_2$ und $R_3$ die in Anspruch 1 oder 2 angegebene Bedeutungen haben, als Wirkstoff in Kombination mit einem inerten, agrikulturell brauchbaren Hilfsstoff.

11. Schädlingsbekämpfungsmittel nach Anspruch 10, dadurch gekennzeichnet, daß es sich bei dem Wirkstoff um eine Verbindung gemäß Anspruch 3 handelt.

**Patentansprüche für den Vertragsstaat: AT**

1. Verfahren zur Bekämpfung von Zecken und/oder Milben, dadurch gekennzeichnet, daß man die Insekten oder Ektoparasiten oder ihre Wirtstiere oder ihren Lebensraum kontaktiert bzw. behandelt mit einer Verbindung der Formel:

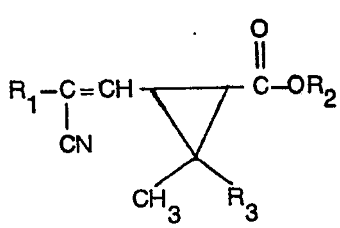

–(I)

wobei $R_1$ für 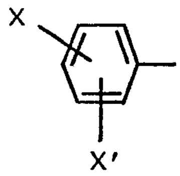, 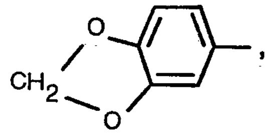, 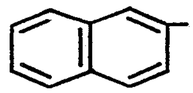 oder 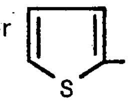 steht;

$R_2$ für eine Einheit 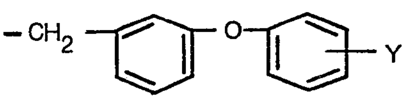 oder 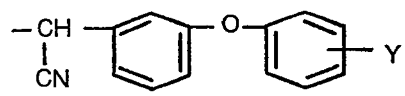

steht, $R_3$ für Wasserstoff oder Methyl steht; X und X′ jeweils Wasserstoff, Halogen, $C_1$—$C_3$-Alkyl, $C_1$—$C_3$-Alkoxy oder Halogenalkyl-$C_1$—$C_2$ bedeuten und Y für Wasserstoff, Halogen, Methyl oder Methoxy steht; und zwar in Mengen, die ausreichen, um ein Bekämpfung der Zecken und/oder Milben zu erreichen.

2. Verfahren nach Anspruch 1, dadurch gekennzeichnet, daß $R_1$ für

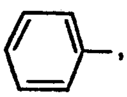, 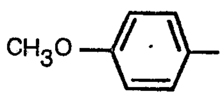, 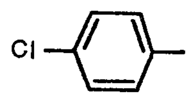, 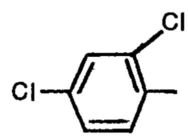, 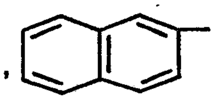 oder 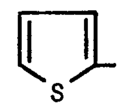;

$R_2$ für 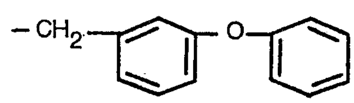 oder 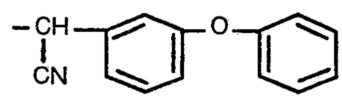

steht; und $R_3$ für Methyl steht.

3. Verfahren nach Anspruch 2, dadurch gekennzeichnet, daß es sich bei der Verbindung handelt um: (*Z*) - *trans* - 3 - (*β* - Cyanostyryl) - 2,2 - dimethylcyclopropancarbonsäure, *α* - Cyano - *m* - phenoxybenzylester; (*Z*) - *trans* - 3 - (*p* - Chlor - *β* - cyanostyryl) - 2,2 - dimethyl-cyclopropancarbonsäure, *m* - Phenoxybenzylester; (*Z*) - *trans* - 3 - (*p* - Chlor - *β* - cyanostyryl) - 2,2 - dimethylcyclopropancarbonsäure, *α* - Cyano - *m* - phenoxybenzylester; (*Z*) - *trans* - 3 - (*β* - Cyano - *p* - methoxystyryl) - 2,2 - dimethylcyclopropancarbonsäure, *m* - Phenoxybenzylester; (*Z*) - *trans* - 3 (*β* - Cyano - p - methoxystyryl) - 2,2 - dimethylcyclopropancarbonsäure, *α* - Cyano - *m* - phenoxybenzylester; (*Z*) - *trans* - 3 - (2,4 - Dichlor - *β* - cyanostyryl) - 2,2 - dimethylcyclopropancarbonsäure, *m* - Phenoxybenzylester; oder (*Z*) - *trans* - 3 - (2,4 - Dichlor - *β* - cyanostyryl) - 2,2 - dimethylcyclopropancarbonsäure, *α* - Cyano - *m* - phenoxybenzylester.

4. Verfahren nach einem der vorstehenden Ansprüche, dadurch gekennzeichnet, daß die Verbindung mit einer Rate von 0,001 ppm bis 100 ppm appliziert wird.

5. Verfahren zur Herstellung einer Verbindung der Struktur gemäß Formel (I):

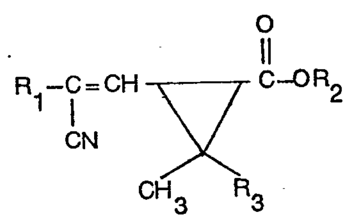

(I)

wobei $R_1$ für 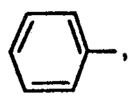, 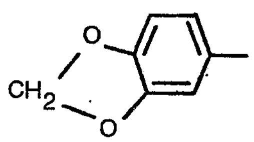, 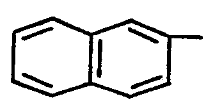 oder 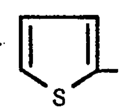 steht;

$R_2$ für eine Einheit 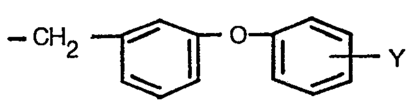 oder 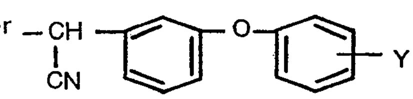

steht; $R_3$ für Wasserstoff oder Methyl steht und Y für Wasserstoff, Halogen, Methyl oder Methoxy steht, dadurch gekennzeichnet, daß man einen Aldehyd der Formel (III):

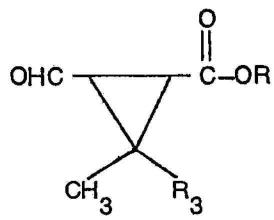

(III),

wobei R für $C_1$—$C_3$-Alkyl steht und $R_3$ die oben angegebene Bedeutung hat, mit einer Verbindung mit einer aktiven Methylengruppe gemäß der Formel $R_1$—$CH_2$—CN, wobei $R_1$ die oben angegebene Bedeutung hat, in Gegenwart einer Base wie Natrium- oder Kaliumäthoxid und einem niederen $C_1$—$C_3$-Alkohol kondensiert, um eine Cyclopropancarbonsäure der Formel (IV) zu erhalten:

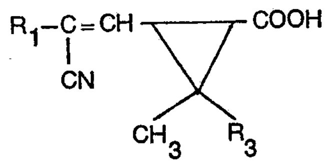

(IV)

die Carbonsäure (IV) in das korrespondierende Säurechlorid (V):

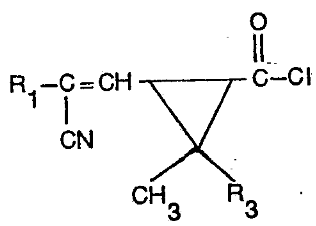

(V)

mit Thionylchlorid in Gegenwart eines aromatischen Lösungsmittels wie Benzol, Toluol oder Xylol umwandelt und das Säurechlorid (V) mit einem Alkohol der Formel:

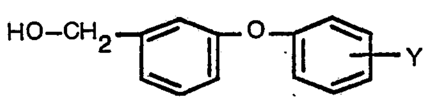 oder 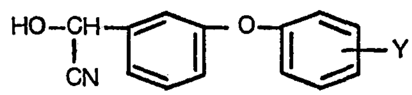

wobei Y die oben angegebene Bedeutung hat, in Gegenwart von Pyridin und einem inerten aromatischen Lösungsmittel, ausgewählt unter Benzol, Toluol und Xylol, umsetzt, um die Verbindung der Formel (I) zu erhalten.

6. Verfahren nach Anspruch 5, dadurch gekennzeichnet, daß $R_1$ für

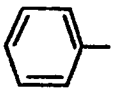, 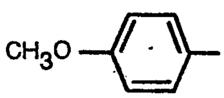, 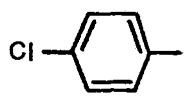, 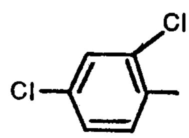, 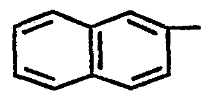 oder 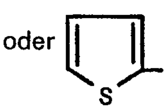;

$R_2$ für 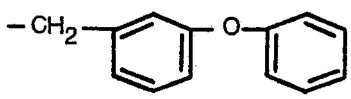 oder 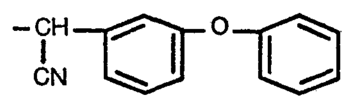

steht; und $R_3$ für Methyl steht; oder ein *cis*- oder *trans*-Isomeres derselben oder ein Isomerengemisch derselben.

7. Schädlingsbekämpfungsmittel, gekennzeichnet durch eine Verbindung der Formel:

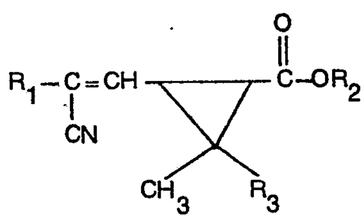

(I)

wobei $R_1$, $R_2$ und $R_3$ die in Anspruch 1 oder 2 angegebenen Bedeutungen haben, als Wirkstoff in Kombination mit einem inerten, agrikulturell brauchbaren Hilfsstoff.

8. Schädlingsbekämpfungsmittel nach Anspruch 7, dadurch gekennzeichnet, daß der Wirkstoff eine Verbindung gemäß Anspruch 3 ist.

9. Verfahren zur Herstellung eines Schädlingsbekämpfungsmittels, dadurch gekennzeichnet, daß man eine Verbindung der Formel:

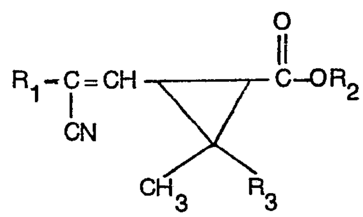 (I)

wobei $R_1$, $R_2$ und $R_3$ die in Anspruch 1 oder 2 angegebenen Bedeutungen haben, einem inerten Agrikultur-Hilfsstoff einverleibt.

**Revendications pour les Etats contractants: BE CH DE FR GB IT NL SE**

1. Un procédé pour lutter contre leas tiques qui comprend la mise en contact des insectes ou des ectoparasites, ou l'application à leurs hôtes ou à leur habitat, d'un composé de formule:

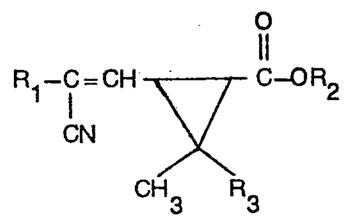 –(I)

dans laquelle $R_1$ est 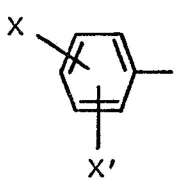, 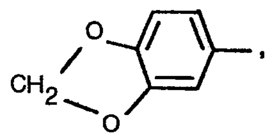, 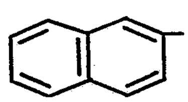 ou 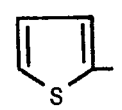;

$R_2$ est un fragment 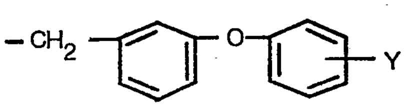 ou 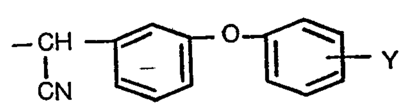

$R_3$ est l'hydrogène ou méthyle; X et X' sont chacun l'hydrogène, un halogène, un alkyle en $C_1$—$C_3$, un alcoxy en $C_1$—$C_3$ ou un halogénoalkyle en $C_1$—$C_2$; Y est l'hydrogène, un halogène, méthyle ou méthoxy; en des quantités suffisantes pour lutter contre les tiques.

2. Un procédé selon la revendication 1, dans lequel $R_1$ est

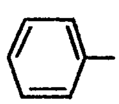, 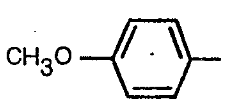, 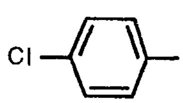, 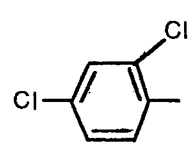, 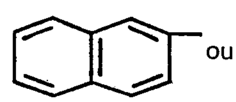 ou 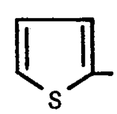;

$R_2$ est 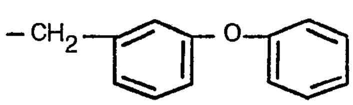 ou 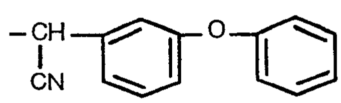;

et $R_3$ est méthyle.

3. Un procédé selon la revendication 2, dans lequel le composé est l'ester $\alpha$ - cyano - m - phénoxybenzylique de l'acide (Z) - trans - 3 - ($\beta$ - cyanostyryl) - 2,2 - diméthylcyclopropanecarboxylique; l'ester m - phénoxybenzylique de l'acide (Z) - trans - 3 - (p - chloro - $\beta$ - cyanostyryl) - 2,2 - diméthylcyclopropanecarboxylique; l'ester $\alpha$ - cyano - m - phénoxybenzylique de l'acide (Z) - trans - 3 - (p - chloro - $\beta$ - cyanostyryl) - 2,2 - diméthyl - cyclopropanecarboxylique; l'ester m - phénoxybenzylique de l'acide (Z) - trans - 3 - ($\beta$ - cyano - p - méthoxystyryl) - 2,2 - diméthylcyclopropane-

carboxylique; l'ester $\alpha$ - cyano - m - phénoxybenzylique de l'acide (Z) - trans - 3 - ($\beta$ - cyano - p - méthoxystyryl) - 2,2 - diméthylcyclopropanecarboxylique; l'ester m - phénoxybenzylique de l'acide (Z) - trans - 3 - (2,4 - dichloro - $\beta$ - cyanostyryl) - 2,2 - diméthylcyclopropanecarboxylique; ou l'ester $\alpha$ - cyano - m - phénoxybenzylique de l'acide (Z) - trans - 3 - (2,4 - dichloro - $\beta$ - cyanostyryl) - 2,2 - diméthylcyclopropanecarboxylique.

4. Un procédé selon l'une quelconque des revendications précédentes, dans lequel le composé est appliqué à raison de 0,001 ppm à 100 ppm.

5. Un composé de formule

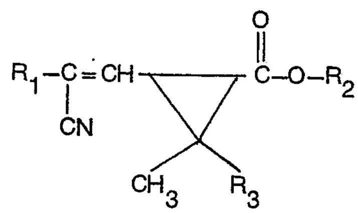

dans laquelle $R_1$ est 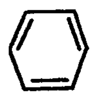, 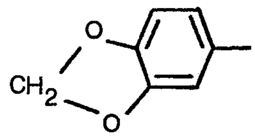, 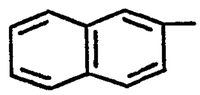 ou 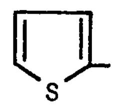;

$R_2$ est 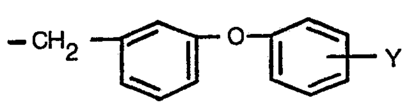 ou 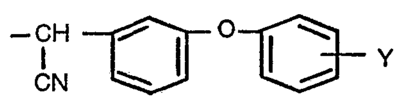

$R_3$ est l'hydrogène ou méthyle; et Y est l'hydrogène, un halogène, méthyle ou méthoxy.

6. Un composé selon la revendication 5 dans lequel $R_1$ est

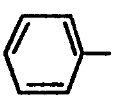 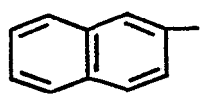 ou 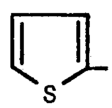;

$R_2$ est 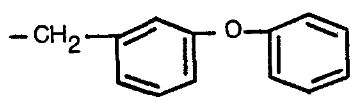 ou 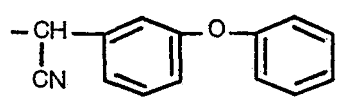;

et $R_3$ est méthyle.

7. Composé selon la revendication 5: l'ester $\alpha$ - cyano - m - phénoxybenzylique de l'acide (Z) - trans - 3 - ($\beta$ - cyanostyryl) - 2,2 - diméthyl - cyclopropanecarboxylique; l'ester m - phénoxybenzylique de l'acide (Z) - trans - 3 - ($\beta$ - cyanostyryl) - 2,2 - diméthylcyclopropanecarboxylique; l'ester $\alpha$ - cyano - m - phénoxybenzylique de l'acide (Z) - trans - 3 - [2 - cyano - 2 - (2 - naphtyl)vinyl] - 2,2 - diméthyl - cyclopropanecarboxylique ou l'ester $\alpha$ - cyano - m - phénoxybenzylique de l'acide (E) - trans - 3 - [2 - cyano - 2 - (2 - thiényl)] - 2,2 - diméthyl - cyclopropanecarboxylique.

8. Un procédé de préparation d'un composé dont la structure est représentée par la formule (I)

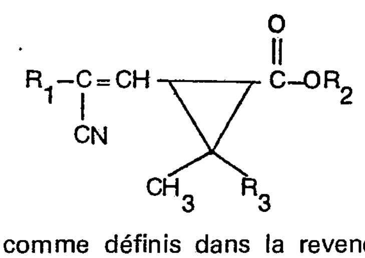

(I)

dans laquelle $R_1$, $R_2$ et $R_3$ sont comme définis dans la revendication 5, caractérisé en ce qu'on condense un aldéhyde de formule (III):

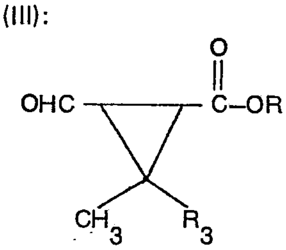

(III),

dans laquelle R est un alkyle en $C_1$—$C_3$ et $R_3$ est tel que défini ci-dessus, avec un composé ayant un groupe méthylene actif et représente par la formule $R_1$—$CH_2$—CN dans laquelle $R_1$ est tel que défini ci-dessus, en présence d'une base telle que l'éthylate de sodium ou de potassium et d'un alcool inférieur en $C_1$—$C_3$, pour obtenir un acide cyclopropanecarboxylique de formule (IV):

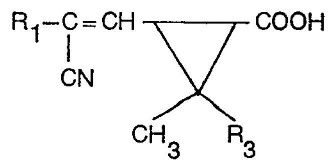

(IV)

ou transforme l'acide carboxylique (IV) en le chlorure d'acide (V) correspondant:

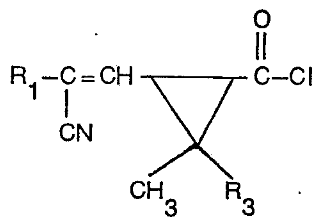

(V)

avec du chlorure de thionyle en présence d'un solvant aromatique constitué de benzène, de toluène ou de xylène, et on fait réagir le chlorure d'acide (V) avec un alcool de formule:

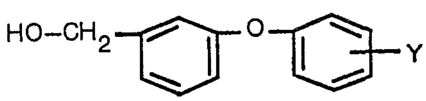 ou 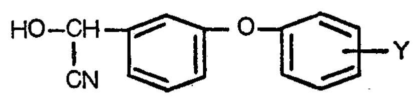

dans laquelle Y est comme défini ci-dessus, en présence de pyridine et d'un solvant aromatique inerte choisi parmi le benzène, le toluène et le xylène pour obtenir le composé de formule (I).

9. Un procédé selon la revendication 8 dans lequel $R_1$ est

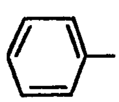 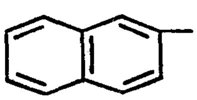 ou 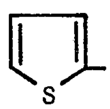 ;

$R_2$ est 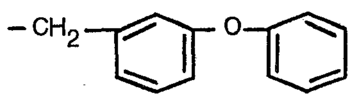 ou 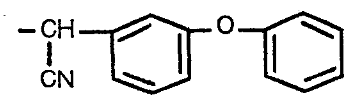 ;

et $R_3$ est méthyle; ou un isomère cis ou trans correspondant ou un mélange isomère correspondant.

10. Composition contre les tiques comprenant un composé de formule:

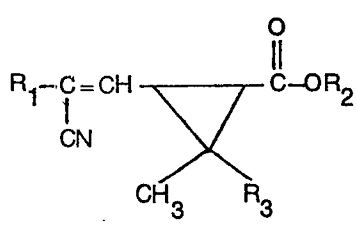

(I)

dans laquelle $R_1$, $R_2$ et $R_3$ sont tels que définis à la revendication 1 ou la revendication 2 comme ingrédient actif, en association avec un adjuvant agricole inerte.

11. Composition contre les tiques selon la revendication 10 dans laquelle le composé actif est tel que défini à la revendication 3.

**Revendications pour l'Etat contractant: AT**

1. Un procédé pour lutter contre les tiques qui comprend la mise en contact des insectes ou des ectoparasites, ou l'application à leurs hôtes ou à leur habitat, d'un composé de formule:

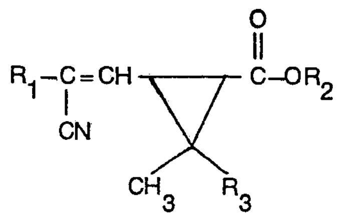
—(I)

dans laquelle $R_1$ est 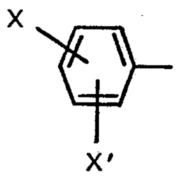, 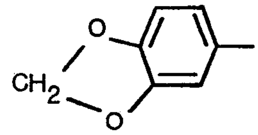, 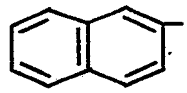 ou 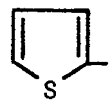;

$R_2$ est un fragment 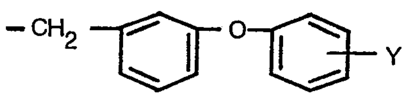 ou 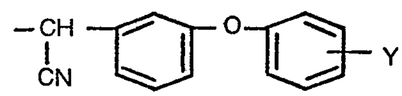

$R_3$ est l'hydrogène ou méthyle; X et X' sont chacun l'hydrogène, un halogène, un alkyle en $C_1$—$C_3$, un alcoxy en $C_1$—$C_3$ ou un halogénoalkyle en $C_1$—$C_2$; Y est l'hydrogène, un halogène, méthyle ou méthoxy; en des quantités suffisantes pour lutter contre les tiques.

2. Un procédé selon la revendication 1, dans lequel $R_1$ est

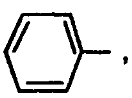, 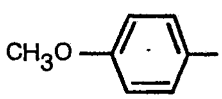, 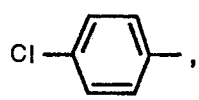, 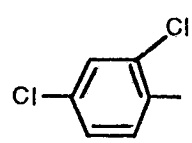, 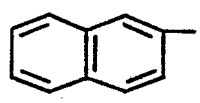 ou 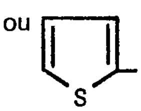;

$R_2$ est 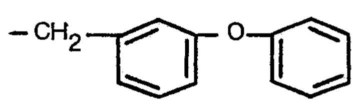 ou 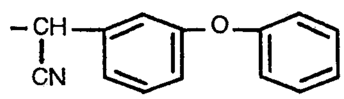;

et $R_3$ est méthyle.

3. Un procédé selon la revendication 2, dans lequel le composé est l'ester $\alpha$ - cyano - m - phénoxybenzylique de l'acide (Z) - trans - 3 - ($\beta$ - cyanostyryl) - 2,2 - dimémethylcyclopropanecarboxylique; l'ester m - phénoxybenzylique de l'acide (Z) - trans - 3 - (p - chloro - $\beta$ - cyanostyryl) - 2,2 - diméthylcyclopropanecarboxylique; l'ester $\alpha$ - cyano - m - phénoxybenzylique de l'acide (Z) - trans - 3 - (p - chloro - $\beta$ - cyanostyryl) - 2,2 - diméthylcyclopropanecarboxylique; l'ester m - phénoxybenzylique de l'acide (Z) - trans - 3 - ($\beta$ - cyano - p - méthostyryl) - 2,2 - diméthylcyclopropanecarboxylique; l'ester $\alpha$ - cyano - m - phénoxybenzylique de l'acide (Z) - trans - 3 - ($\beta$ - cyano - p - méthoxystyryl) - 2,2 - diméthylcyclopropanecarboxylique; l'ester m - phénoxybenzylique de l'acide (Z) - trans - 3 - (2,4 - dichloro - $\beta$ - cyanostyryl) - 2,2 - diméthylcyclopropanecarboxylique; ou l'ester $\alpha$ - cyano - m - phénoxybenzylique de l'acide (Z) - trans - 3 - (2,4 - dichloro - $\beta$ - cyanostyryl) - 2,2 - dimémethylcyclopropanecarboxylique.

4. Un procédé selon l'une quelconque des revendications précédentes, dans lequel le composé est appliqué à raison de 0,001 ppm à 100 ppm.

5. Un procédé de préparation d'un composé dont la structure est représentée par la formule (I):

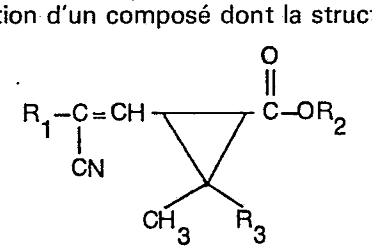
—(I)

dans laquelle $R_1$ est 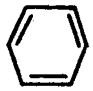 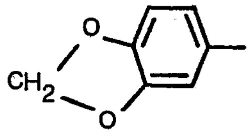, 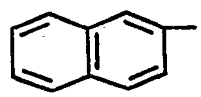 ou 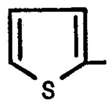;

$R_2$ est un fragment 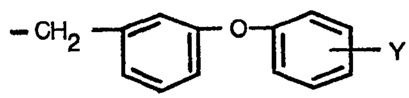 ou 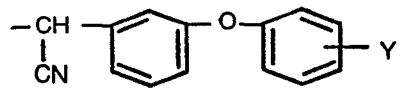

$R_3$ est l'hydrogène ou méthyle; et Y est l'hydrogène, un halogène, méthyle ou méthoxy; caractérisé en ce qu'on condense un aldéhyde de formule (III):

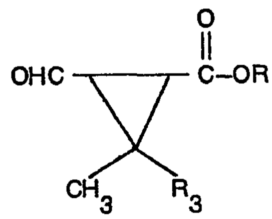

(III),

dans laquelle R est un alkyle en $C_1$—$C_3$ et $R_3$ est tel que défini ci-dessus, avec un composé ayant un groupe méthylène actif et représenté par la formule $R_1$—$CH_2$—CN dans laquelle $R_1$ est tel que défini ci-dessus en présence d'une base, telle que l'éthylate de sodium ou de potassium, et d'un alcool inférieur en $C_1$—$C_3$ pour obtenir un acide cyclopropanecarboxylique de formule (IV):

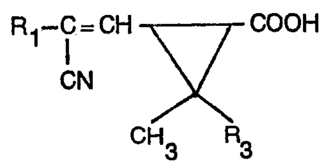

(IV)

on transforme l'acide carboxylique (IV) en le chlorure d'acide (V) correspondant:

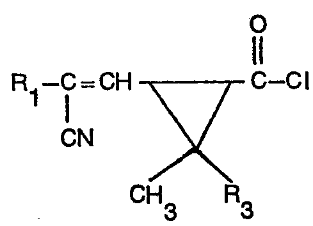

(V)

avec du chlorure de thionyle en présence d'un solvant aromatique constitué de benzène, de toluène ou de xylène, et on fait réagir le chlorure d'acide (V) avec un alcool de formule:

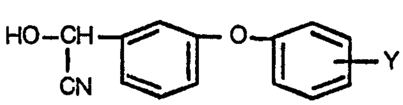 ou 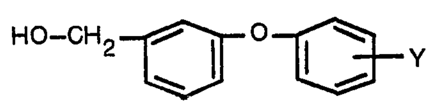

dans laquelle Y est tel que défini ci-dessus, en présence de pyridine et d'un solvant aromatique inerte choisi parmi le benzène, le toluène et le xylène pour obtenir le composé de formule (I).

6. Un procédé selon la revendication 5 dans lequel $R_1$ est

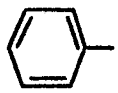, 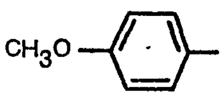, 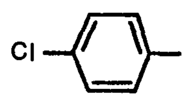, 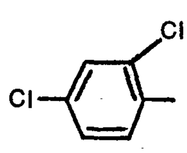, 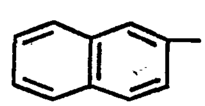 ou 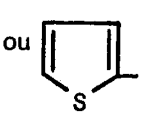;

$R_2$ est 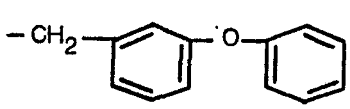 ou 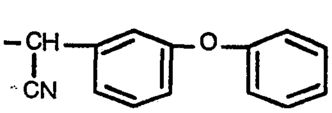;

et $R_3$ est méthyle; ou un isomère cis ou trans correspondant ou un mélange isomère correspondant.

7. Composition contre les tiques comprenant un composé de formule:

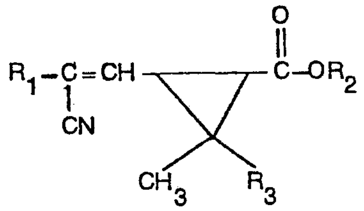

(I)

dans laquelle $R_1$, $R_2$ et $R_3$ sont tels que définis à la revendication 1 ou à la revendication 2 comme ingrédient actif, en association avec un adjuvant agricole inerte.

8. Composition contre les tiques selon la revendication 7 dans laquelle ledit composé actif est tel que défini à la revendication 3.

9. Un procédé de préparation d'une composition contre les tiques caractérisée en ce qu'on incorpore un composé de formule:

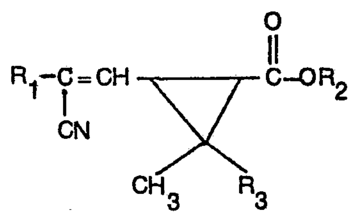

(I)

dans laquelle $R_1$, $R_2$ et $R_3$ sont tels que définis à la revendication 1 ou à la revendication 2, avec un adjuvant agricole inerte.